# EUROPEAN PATENT SPECIFICATION

(11) **EP 3 427 781 B1**
(45) Date of publication and mention of the grant of the patent: **25.09.2024**
(21) Application number: 18181042.5
(22) Date of filing: 30.06.2018
(51) Int. Cl.: A61M 11/00, A61J 1/10, A61J 1/14, A61J 1/20, B65D 81/32, A61M 13/00

(54) **DRUG AND DEVICE SYSTEM FOR PRESSURIZED AEROSOL THERAPIES INTO A MAMMALIAN HOLLOW SPACE**
ARZNEIMITTEL UND VORRICHTUNGSSYSTEM FÜR DRUCKBEAUFSCHLAGTE AEROSOLTHERAPIEN IN EINEM SÄUGETIERHOHLRAUM
MÉDICAMENT ET SYSTÈME DE DISPOSITIF POUR DES THÉRAPIES D'AÉROSOL SOUS PRESSION DANS UN ESPACE CREUX D'UN MAMMIFÈRE

(30) Priority: 11.07.2017 EP 17180775
(43) Date of publication of application: 16.01.2019
(73) Proprietor: Pharma Resources GmbH, 31785 Hameln (DE)
(72) Inventor: Luerig, Detlef, 31863 Coppenbruegge (DE)
(74) Representative: Gosmann, Martin

(56) References cited:
- EP-A1- 0 395 758
- EP-A1- 2 399 565
- EP-A1- 2 962 676
- EP-A1- 3 158 987
- WO-A2-2011/071952
- DE-A1- 4 408 498
- GB-A- 2 117 733
- JP-A- 2000 334 042
- US-A- 4 410 321
- US-A- 5 836 359
- US-A1- 2003 100 866
- US-A1- 2009 216 184
- US-A1- 2011 178 495
- US-A1- 2013 006 170
- US-A1- 2013 299 021
- US-A1- 2014 121 589
- US-A1- 2014 142 495
- US-B1- 6 221 041

## Description

The invention relates to a drug and device system for the directed application of a substance into a hollow space, such as a hollow organ, or into a body cavity of a patient, i.e. a mammalian patient, in particular for use in the course of a therapeutic pneumoperitoneum. In particular, herein the present invention is for use in cancer/tumor treatment, including metastatic forms thereof, and is also directed to a kit and its use in a method of treatment, comprising the said drug and device system for the said directed application.

The treatment of diseases, particularly of cancer and/or tumors in a hollow space, such as a hollow organ, or into a body cavity of a patient, i.e. a mammalian patient, is complex and systemic treatments are demanding high dosages of substances with undesirable and enormous side-effects., and in case of cancer and/or tumors, for example, in a body cavity of a patient, often even high dose and long-term treatment do not provide sustained diminishing and/or at least sufficient delay of disease progression.

For example, peritoneal carcinomatosis is the most common terminal feature of abdominal cancers. Although the disease is limited to the peritoneal surface, very often a complete surgical removal is impossible and systemic chemotherapy is powerless since such a systemic chemotherapy is performed in most cases as multi-stage chemotherapy, wherein the effectiveness decreases with each stage. Moreover, only a little amount of the systemic chemotherapy reaches the tumor. Thus, peritoneal carcinomatosis is generally considered to be an untreatable condition that makes clinicians abandoning further aggressive treatments.

Peritoneal neoplasia can originate *de novo* from the peritoneal tissues (primary) or invade or metastasize into the peritoneum from adjacent or remote organs (secondary) by lymph- or bloodstream. Primary cancers here are mostly advanced carcinoma of the Colon, Rectum, Ovary and Stomach. In the course of increasing degeneration (mutational oncogenesis) of cancer cells, they eventually lose their ability to hold on to surrounding cells. So it happens that in advanced cancers, individual cells or small groups repeatedly dissolve from the main cell structure of the original tumour (primary tumour). With the blood or lymph stream, sometimes directly (by continuity), then move to other organs to settle, a process defined as metastasis. Especially above-named colon cancer (colon or rectum), ovarian cancer and stomach cancer (gastric cancer) often metastasize into the peritoneum.

From the general state of the art it is well known that in the treatment of diseases in the abdomen and thorax minimally invasive surgery can be performed by means of so-called keyhole surgery, also known as laparoscopy or thoracoscopy. In order to perform keyhole surgery, a hollow space in the patient to be treated is needed. Typically, this hollow space is formed by a gas, preferably carbon dioxide (CO2) being introduced into the patient at a suitable point under a pressure of 12 to 15 mm Hg by means of a CO2-insufflator. Access to the hollow space and for the surgical procedure is provided via operating trocars. Basically it can be said that surgical procedures are still mechanical and/or electrical interventions that do not incorporate other methods of treatment, such as medications. According to the current state of the art during minimally invasive surgery the operating environment to be treated by this procedure is not treated in a controlled way. Only in exceptional cases a therapeutic rinsing with tumor-inhibitory or bactericidal substances is done. The reason for this is that the therapeutic rinsing of the peritoneal cavity had been relatively ineffective. The peritoneum for example forms a barrier that is difficult to overcome. The rinsing solution thus often reaches only a small part of the peritoneal surface. Moreover, the diffusion of the therapeutic solution into the tissue is minimal. The same applies to the thorax and the pleura.

In the recent past, a new treatment concept for treating cancer and/or tumors in, for example, a body cavity of a patient has been developed based on the techniques of laparoscopy, and e.g. on intraperitoneal insufflation or pressurized aerosol (chemo)therapy, respectively. Pressurized aerosol therapy (PAT) is still to be regarded as a new treatment that applies (chemo)therapeutic drugs into the cavity of a patient as an aerosol under pressure. Currently, in the prior art still conventional laparoscopy equipment or devices, and methods, respectively, are used only, for reconstituting and providing the drug, just shortly before its intended application to a patient.

For example, the WO 2009/062740 A1 discloses the use of an ozone/oxygen mixture as primary anticancer therapy through intraperitoneal insufflation. Squamous cell carcinomas of the head and neck region (HNSCC) form a group of metastasizing tumors with a high mortality rate in humans and animals. Since it has been discovered that the biomolecule ozone inhibits the growth of various carcinoma cells in vitro, in the WO 2009/062740 A1 the highly aggressive and lethal VX2 carcinoma of the New Zealand rabbit is used as HNSCC tumor model in order to test whether ozone has antitumor effects in vivo. Therapeutic insufflation of a medical ozone/oxygen (O3/O2) gas mixture into the peritoneum (O3/O2 pneumoperitoneum) at an advanced stage of a neoplastic disease was investigated for the survival rate of rabbits, tumor regression and the absence of local or remote pulmonary metastases. Although the exact mechanisms of action are still unclear, it is concluded that the available data indicates that O3/O2 pneumoperitoneum is a promising new strategy in tumor therapy.

The DE 10 2013 109896 A1 relates to a device suitable for introduction and use within a body cavity, in particular a hollow organ of a patient, wherein the device is comprising a) a first expandable fluid chamber for sealing a first section of the body cavity, b) an aerosol generating device for producing a therapeutic aerosol in a treatment section of the body cavity which extends after the said first section of the body cavity, c) an insufflating device for insufflating a gaseous component (CO2, O2) into the treatment section of the body cavity. The DE 10 2013 109896 A1 concerns laparoscopy equipment or devices adapted for administering a therapeutic aerosol, but is silent about the reconstituting and providing the drug before the therapeutic aerosol generation and its application to a patient.

The US 2014/142495 A1 relates to a surgical device for use in laparoscopy, wherein the device is for the directed application of a substance (X) in a hollow space, such as a hollow organ, of a body cavity, in particular a therapeutic pneumoperitoneum, comprising: a trocar system with a trocar sleeve, said trocar system having a gas connection to which an insufflation gas-supply line can be connected, and a nozzle system, which in its interior forms a lumen, with a proximal end and a distal end, wherein the nozzle system has a needle nozzle fixed at the distal end with the lumen and is guided by the trocar sleeve. However, the US 2014/142495 A1 is silent about the reconstituting and providing the drug before the therapeutic aerosol generation and its application to a patient.

The US 2009/216184 A1 is directed to a drug storage and delivery device, wherein the drug storage and delivery device is comprising a reservoir containing a liquid, a vial containing a dry drug, such as a lyophilized drug, means (M) for forcing the liquid from the reservoir to the vial, via an established fluid connection, and locking means adapted to prevent the means (M) for establishing a fluid connection from re-establishing a previously disconnected fluid connection between the reservoir and the vial. Thereby the dry drug is reconstituted, and the forcing means (M) may subsequently be used for forcing reconstituted drug out of the device. The reservoir, the vial, the fluid connection and the forcing means (M) may form an at least substantially integral unit. Thereby, the number of steps needed to be performed by the user is reduced, the risk of contamination is reduced, the risk of incorrect reconstitution and dosage is reduced, and the device is easy to operate, e.g. using just one hand. However, the US 2009/216184 A1 does not make reference to any pressurized aerosol therapies (PAT) which are subject of the present invention.

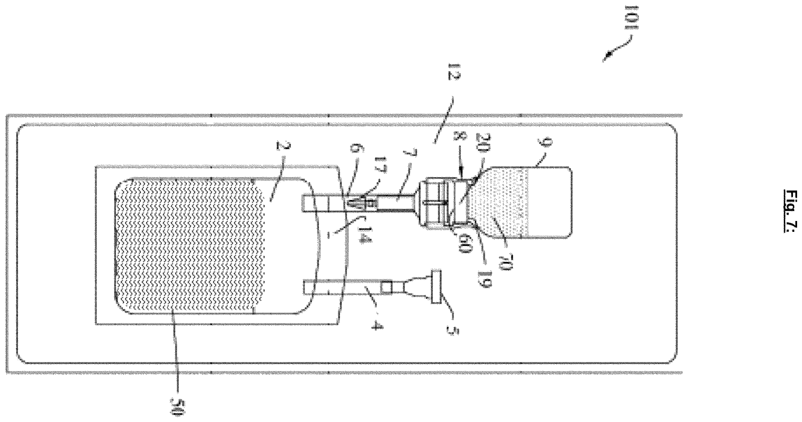

The EP 2962676 A1 is directed to a flexible package with sealed sterile chamber for the reconstitution and administration of fluid medicinal or nutritional substances instillable into the body of a patient. For the meaning of the reference signs, refer to Fig. 6 and Fig. 7 as appended in the drawing section of this present invention disclosure. Thus, the EP 2962676 A1 discloses a package (100, 101) for infusion or instillation of medicinal or nutritional products into the body of a patient is described, comprising a bag (2) of liquid diluent (50) equipped with at least one drain tube (4), and a mixing tube (6) equipped with a coupling and perforation device (8) for a bottle (9) of a pharmacological or nutritional substance (70) in powder, gel or other material, provided with a perforable cap (60). The package (100, 101) comprises a flexible airtight sterile casing (12), containing said bottle (9) of the substance in powder or other and the coupling and perforation device (8). The bottle (9) is housed in the casing (12) in a coupling position with the coupling and perforation device (8) and is manually maneuverable from the outside of the casing (12) up to a perforating position of the cap of the bottle (9). Further, the EP 3158987 A1 discloses a related sterilizable flexible package for the reconstitution and administration of fluid medicinal or nutritional substances which are infused or instillable within the body of a patient and process for the sterilization thereof. In the EP 3158987 A1 it is described a package for the reconstitution and administration of fluid medicinal or nutritional substances which are infused or instillable within the body of a patient, comprising a flexible casing containing a vial of a medicinal or nutritional substance in a coupling position to a coupling and perforation device inserted into a mixing tube for the connection with a bag of liquid diluent. The casing comprises a connector provided with an openable and hermetically closable cap, adapted to introduce a mixture of sterilizing gas and oxygen into said casing. Both, the EP 2962676 A1 and EP 3158987 A1, only disclose flexible packages for use in infusion and instillation treatments. However, they do not make reference to any pressurized aerosol therapies (PAT) which are subject of the present invention.

Another closed drug delivery system is described the patent specification of US 4410321. A closed system is described for separately storing and selectively mixing two components, such as a drug and a diluent, under sterile conditions. The closed system can incorporate a drug vial of standard construction. A unique junction of the closed system permits a drug vial with a drug therein to be connected in a sterile manner with a compressible chamber having a diluent therein, after the drug and diluent have been separately sterilized. The junction encloses the end portions of access means to each of the receptacles, maintaining the end portions in sterile, spaced relation and providing for the selective establishment of a sterile pathway between the drug and diluent for mixing in a closed environment. The compressible chamber is of a unique design including gas-trapping and reservoir compartments in open communication. The compressible chamber is utilized with the junction to provide an efficient, sterile storage and mixing system. However, the US 4410321 does not make reference to any pressurized aerosol therapies (PAT) which are subject of the present invention.

Finally, the GB 2117733 A discloses a sterilized system for mixing liquid and powder includes a first container for holding liquid and another attached container for holding dry solid material. A sealed connection between the said containers is breakable to permit mixing whilst maintaining the said container interiors sealed from the atmosphere. At least the first container and the connection are sealed in an overpouch to protect the container and to reduce water vapor loss through the container walls. Sterilized liquid can be admitted to the said first container from still a further container via a tube which passes through the sealed periphery of the said overpouch. However, the GB 2117733 A does not make reference to any pressurized aerosol therapies (PAT) which are subject of the present invention.

The Pressurized aerosol therapies (PAT) recently introduced at still yet a few hospitals are applied only since about 3-4 years, including university clinics and cancer centers from Germany, France, Denmark, Italy, Australia, Russia, Brazil and Switzerland have started a clinical PIPAC research program. In Germany, presently there are three hospitals of the maximum care to treat the first patients.

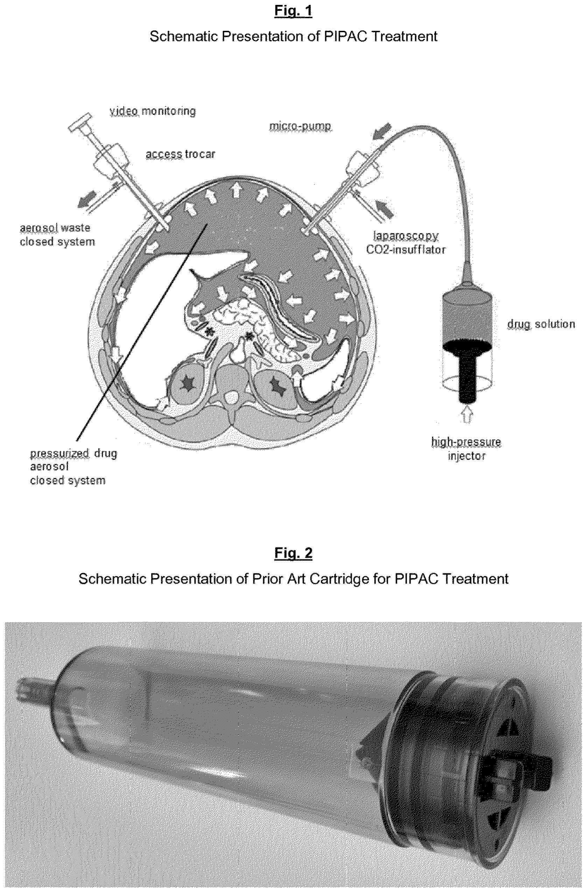

Although, in the prior art, for example as cited above (e.g., EP 2962676 A1 or EP 3158987 A1), packages for intravenous infusions or instillations are known, which comprise a flexible bag in which a chamber for containing a diluent is formed, and from which a flexible tube with an openable closure extends, equipped with a coupling and perforation device, through which a pharmaceutical or nutritional substance in powder, gel or other forms is withdrawn from a bottle and inserted into the inner chamber of the bag, which substance, once mixed with the diluent, forms the medicament or nutrient to be supplied to the patient, at present, however, each dosage to be administered by the new pressurized aerosol therapies (PAT) must still be individually prepared, e.g. reconstituted, by a (hospital) pharmacy under (more or less) sterile and environmental conditions. The prepared, e.g. reconstituted, drug solution is then transferred through the (hospital) pharmacy into a kind of cartridge from the area of radiology (see Fig. 2), which in turn then must be delivered under (more or less) sterile and environmental conditions to the surgical room, where finally by means of a corresponding pump (also from the area of radiology) and nozzle it is administered into the patient by the new pressurized aerosol therapies (PAT). The entire procedure of individually preparing, e.g. reconstituting, and delivering to the and the administration of the said cytostatic substances is off-label and carries a known risk of unwanted exposure to the cytotoxic substances, to the environment, e.g. contaminating the (hospital) pharmacy, the hospital, the surgical room etc., and in particular known risk of unwanted exposure to the concerned (hospital) pharmacy staff and/or physicians, healthcare assistants, pharmacists, clinicians, surgeons, nurses and potentially other staff.

Thus, it is an object of the present invention to provide a simplified drug and device system for the directed application of a substance by means of a pressurized aerosol therapy (PAT) into a hollow space, such as a hollow organ, or into a body cavity of a patient, i.e. a mammalian patient, in particular for use in the course of a therapeutic pneumoperitoneum. Herein, it is a further object of the present invention to provide such a simplified drug and device system for the directed application of a substance by means of a pressurized aerosol therapy (PAT), which is for use in cancer/tumor treatment, including metastatic forms thereof, and in still another object to provide a kit and its use in a method of treatment, comprising the said drug and device system for the said directed application of a substance by means of a pressurized aerosol therapy (PAT). The objects of the present invention include that drug and device system for the directed application of a substance by means of a pressurized aerosol therapy (PAT) wherein the said drug and device (drug containing (medical) device) system is prefilled and is in compliance with the guidelines of a competent drug regulatory authority applicable for pre-filled systems, preferably for pre-filled systems for long-term storage.

These objects are solved with the features of the independent claim.

Further exemplary embodiments are specified in the dependent claims which refer back to said independent claim.

Accordingly, in an embodiment, the present invention, as defined in the any of the claims and as further described herein, is directed to a simplified drug and device system for the directed application of a substance by means of a pressurized aerosol therapy (PAT) into a hollow space, such as a hollow organ, or into a body cavity of a patient, i.e. a mammalian patient, in particular for use in the course of a therapeutic pneumoperitoneum. In another embodiment, the present invention, as defined in the any of the claims and as further described herein, is directed to such a simplified drug and device system for the directed application of a substance by means of a pressurized aerosol therapy (PAT), which is for use in cancer/tumor treatment, including metastatic forms thereof. In still another embodiment, the present invention, as defined in the any of the claims and as further described herein, is directed to a kit and its use in a method of treatment, comprising the said drug and device system for the said directed application of a substance by means of a pressurized aerosol therapy (PAT). In a further embodiment, the present invention, as defined in the any of the claims and as further described herein, is directed to such a drug and device system for the directed application of a substance by means of a pressurized aerosol therapy (PAT) wherein the said drug and device (drug containing (medical) device) system is prefilled and is in compliance with the guidelines of a competent drug regulatory authority applicable for pre-filled systems, preferably for pre-filled systems for long-term storage.

Pressurized aerosol (chemo)therapy (PAT), for example but not limited to, pressurized intraperitoneal aerosol chemotherapy (PIPAC) is still to be regarded as a new treatment that applies chemotherapeutic drugs into the peritoneal cavity as an aerosol under pressure. Benefits of PIPAC compared to HIPEC (intraperitoneal hyperthermic chemotherapy) treatment known in the prior art, or conventional systemic chemotherapy are an improved local bioavailability of chemotherapeutic drugs. Due to a decreased dose the patients suffer from less negative side effects and also risks of contamination (e.g. by extravasa-tion during injection or infusion) and unexpected exposure (e.g. by accident) of both patients and health care professionals are minimized. The surgical, laparoscopy intervention is minimal: A trocar is passed through the abdominal wall. This is an instrument that allows access to a body cavity by minimally invasive surgery (e.g. abdomen, chest cavity). Through this approach, the abdominal cavity is then filled with CO2, so that the abdominal cavity is inflated. The inflated abdominal cavity can now be entered with a video camera to observe the intervention. Subsequently, a second trocar is inserted at another point in the abdominal wall. Samples of tumour tissue are taken for analysis. This is followed by the actual PIPAC treatment: An application lance is inserted into the abdominal cavity through the second trocar. At this time, leak testing is performed to assure that no cytostatics may leave into the environment during the pressurized injection. The cytostatics are sprayed inside the abdominal cavity by a pump system, normally used for scintigraphy (injection of radioactive materials). The calculation of the dose for the used cytostatic drugs is carried out according to the same principles of a systemic intravenous therapy, but only 10% of the systemic dose may be used. After application, followed by a reaction time of about 30 minutes, the aerosol is removed by gas extraction, followed by the removal of operational equipment in combination with discharging gas from the abdominal cavity.

The same advantages apply to other pressurized aerosol (chemo)therapies (PATs), for example, besides the before already mentioned pressurized intra-peritoneal aerosol chemotherapy (PIPAC), for example but not limited to, also to pressurized intra-thoracic aerosol chemotherapy (PITAC), pressurized intra-luminal aerosol chemotherapy (PILAC), and pressurized intra-vesical aerosol chemotherapy (PIVAC), pressurized intra-rectal aerosol chemotherapy (PIRAC).

Thus, complementing the search for novel cancer therapies that can be used in conjunction with existing treatments, a new and advantageous "ready-to use" drug and device system for the directed application of a substance by means of a pressurized aerosol therapy (PAT), or a "ready-to use" kit comprising the said drug and device system of the present invention, which avoids the current disadvantages and risks of the prior art.

### Terms as used herein in the context of the invention:

The term "prefilled" denotes the prefilling of a drug-container with a pharmaceutically active substance and/or of pharmaceutically acceptable diluent bags in a pharmaceutical industry manner and as approved by a drug regulatory authority, especially for safety reasons (e.g. concerning the medication and or potential contamination and/or toxification risks), with protection for long-term, and low-cost maintenance. Thus the invention is directed to a drug substance, i.e. a pharmaceuically active substance, contained in a drug/device system (drug device system), i.e. in a drug containing device system and/or in a drug containing medical device system, and a pressurized aerosol method utilizing a drug storage container prefilled pharmaceutically or in a pharmaceutical compounding facility with a drug which system or method involves long-term stability of the drug prefilled in a drug storage container and of a liquid diluent in a separate liquid diluent storage bag, thus enabling the simplification of both, the providing of the drug substance for a particular application and the hospital treatment procedures. In particular, the system and method comprises preferably a label/marking, providing information in a visual, readable, barcode and/or QR-code, and/or wireless way in order to avoid medical errors regarding the administration of concerned drugs. The invention preferably concerns a drug/device system (drug device system), i.e. a drug containing device system and/or a drug containing medical device system, for a pressurized aerosol therapy, optionally as a kit wherein a pump and/or an application lance and/or injection lance with nozzle for applying, i.e. for use in administering a drug substance as described herein, is combined together with a package comprising, each separately, a drug-prefilled container and a liquid diluent in a bag, wherein said term "prefilled" has the meaning as commonly used in a pharmaceutical authorization, e.g. the term is meant to define compliance with regulatory requirements, preferably those set forth in the applicable guidelines of the European Medicine Agency (EMA, previously known as EMEA) and more preferably as additionally set forth with the applicable guidelines of the Food & Drug Administration (FDA) in the USA, for a new drug packaging (pharmaceutical envelope) having a longer shelf-life, particularly longer than one, two, three, four, five, six month or even longer up to one year or up to two years, following a filling method in a pharmaceutical compounding facility, particularly designed for the optimal and reliable cooperation between the prefilled drug/device system (drug device system), i.e. drug containing device system and/or drug containing medical device system, and a pressurized aerosol therapy equipment in a hospital, which guarantees greater safety when applying, in particular dangerous or toxic drug substances like for example anticancer or antitumor cytostatic substances. Accordingly, the term "prefilled" means that the drug/device system (drug device system), i.e. drug containing device system and/or drug containing medical device system, is filled with the drug substance in a suitable drug-container, and separately with liquid diluent in a diluent-bag, by a pharmaceutical company, in such way that it can be shelved and/or supplied by conventional pharmaceutical supply chain management. This means, in contrast to the state of the art, that the drug/device system (drug device system), i.e. drug containing device system and/or drug containing medical device system, is not filled in a hospital, clinic or a pharmacy directly prior to individual administration to a patient, but can be produced in an industrial pharmaceutical manner more efficiently and on beforehand. Accordingly, using a prefilled drug/device system (drug device system), i.e. drug containing device system and/or drug containing medical device system,, a compounding method of filling (e.g. by providing a compounder in each country) can offer the desired stability (long time on the shelf) and application safety of drug substance, in particular of anticancer and/or antitumor and/or cytostatic substances, so that storage at hospital can be profitable avoiding safety risks, burden and errors of local filling. Thus, the term "long-term" in the context of the invention is meant to designate a longer shelf-life of the drug-prefilled drug/device system (drug device system), i.e. drug containing device system and/or drug containing medical device system, for pressurized aerosol therapy, of at least 1 month, preferably at least 2 months, more preferably at least 3, 4, 5, 6 months, and most preferably more than one year, especially up to two years, under standard room conditions.

The term "drug" in the context of the invention is understood in broad sense by a person skilled in the art, and denotes any substance, i.e. a pharmaceuically active substance or pharmaceuically active ingredient, for use in a pharmaceutical and/or medical treatment or pharmaceutical and/or medical prevention of a disease and/or disorder, in a subject, in particular in a human subject, Thus, the term "drug" in the context of the invention denotes an active ingredient (Al) that is the ingredient in a pharmaceutical drug and that is biologically active. The similar terms active pharmaceutical ingredient (API) and bulk active are also used in medicine, and the term active substance may be used for natural products.

The term "pressurized aerosol therapy" denotes a new treatment concept for treatments performed in, for example, a body cavity of a patient based on the techniques of laparoscopy, and e.g. on intraperitoneal insufflation or pressurized aerosol (chemo)therapy, respectively. Pressurized aerosol therapy (PAT) is a new treatment that applies (chemo)therapeutic drugs into the cavity of a patient as an aerosol under pressure, for example as disclosed in the DE 10 2013 109896 A1, which relates to a device suitable for introduction and use within a body cavity, in particular a hollow organ of a patient, wherein the device is comprising a) a first expandable fluid chamber for sealing a first section of the body cavity, b) an aerosol generating device for producing a therapeutic aerosol in a treatment section of the body cavity which extends after the said first section of the body cavity, c) an insufflating device for insufflating a gaseous component (CO2, O2) into the treatment section of the hollow organ or body cavity.

The term "insufflation" demotes an act of blowing such as a gas, powder, or vapor into a body cavity. Insufflation has many medical uses, most notably as a route of administration for various drugs, and wherein the term "blowing" requires the application of positive pressure to push the substance into the hollow organ or body cavity. For example, gases are often insufflated into a body cavity to inflate the cavity for more workroom, e.g. during laparoscopic surgery. The most common gas used in this manner is carbon dioxide, because it is non-flammable, colorless and dissolves readily in blood.

The term "efficacy" is the ability to achieve a satisfactory treatment and/or prophylaxis. It is used in pharmacology and medicine to refer to both the maximum response achievable from a pharmaceutical drug in research settings, and to the capacity for sufficient therapeutic effect or beneficial change in clinical settings. In medicine, efficacy is the capacity for beneficial change, or therapeutic effect, of a given intervention, for example, a drug, (medical) device, surgical procedure, or a public health intervention. Establishment of the efficacy of an intervention is often done relative to other available interventions, with which it will have been compared. Specifically, efficacy refers to whether a drug demonstrates a health benefit over a placebo, or conventional/comparison treatment, or other intervention when tested in an ideal situation, such as a tightly controlled clinical trial. These studies focus to a primary parameter to be shown statistically different changes between comparison and intervention groups. A therapeutic effect is a consequence of a treatment of any kind, the results of which are judged to be desirable and beneficial. This applies whether the result was expected, unexpected, or even an unintended consequence of the treatment. An adverse effect, on the other hand, is a harmful and undesired effect. What constitutes a therapeutic effect versus a side effect is a matter of both the nature of the situation in which a treatment is used and the goals of treatment. Those changes which are viewed as desirable, given the situation, are called therapeutic; those undesirable for the situation are viewed as harmful.

As used herein, the term "topical efficacy" or "topical therapeutic efficacy" refers to a topical (therapeutic) effect, in the pharmacodynamic sense, and thus refers to a local, rather than systemic, target for a medication. Accordingly, local efficacy means a local therapy and/or prophylaxis of an active substance specifically or selectively to a location where, for example, the medication shall deliver its direct therapeutic and/or prophylactic effect and does not or only to a low degree enter the circulatory system, e.g., thereby not causing any or only a low systemic action. In this regard, the topical efficacy of the present invention is also contrasted with enteral (in the digestive tract) and intravascular/intravenous (injected into the circulatory system) administrations. In comparison to compositions aiming at systemic availability, the topical efficacy may also be characterized by longer latency times until systemic levels of the active substance(s) increase. Particularly, in the context of the present invention, topical efficacy preferably means that blood and/or plasma and/or serum levels of the active substance and/or metabolites thereof do not exceed levels which are two orders (preferably one order) of magnitude higher than the levels measured in the same person before dosing.

Consequently, a topical treatment is a medication that is applied to a particular place on or in the body, as opposed to systemically. Herein, this means application to internal body surfaces, i.e. or applied to the surface of membranes or tissues of a hollow organ and/or a body cavity. As a route of administration, the topical route is contrasted with the enteral route (in the digestive tract), the intravenous route (injected into veins), and others. A topical effect, in the pharmacodynamic sense, thus refers to a local, rather than systemic, target for a medication. Accordingly, as used herein, the term "topical efficacy" or "topical therapeutic efficacy" also refers to a non-systemic treatment or "non-systemic therapeutic treatment, i.e. as contrasted to a systemic (therapeutic) treatment or drug application, for example by enteral and/or parenteral application, and in particular as contrasted to infusion and/or instillation. The term "systemic" is understood by those skilled in the art to mean systemic (therapeutic) administration which is a route of administration of medication, nutrition or other substance into the circulatory system so that the entire body is affected. Administration can take place via enteral administration (absorption of the drug through the gastrointestinal tract) or parenteral administration (generally injection, infusion, or implantation). Thus, systemic administration is in contrast with topical administration where the effect is generally local.

Drug regulation and drug regulatory authority: The regulation of therapeutic goods, that is drugs and therapeutic devices, varies by jurisdiction. In some countries, such as the United States, they are regulated at the national level by a single agency. In other jurisdictions they are regulated at the state level, or at both, state and national levels by various bodies, as is the case in Australia. The European Medicines Agency (EMA) is a European Union agency for the evaluation of medicinal products. Prior to 2004, it was known as the European Agency for the Evaluation of Medicinal Products (EMEA). The EMA was set up in 1995 in an attempt to harmonize (but not replace) the work of existing national medicine regulatory bodies. As a particular example of such a national medicine regulatory body, reference is made to the Federal Institute for Drugs and Medical Devices (in German: Bundesinstitut für Arzneimittel und Medizinprodukte - BfArM) which is the competent medical regulatory body in Germany. The role of therapeutic goods regulation is designed mainly to protect the health and safety of the population. Regulation is aimed at ensuring the safety, quality, and efficacy of the therapeutic goods which are covered under the scope of the regulation. In most jurisdictions, therapeutic goods must be registered before they are allowed to be marketed. There is usually some degree of restriction of the availability of certain therapeutic goods depending on their risk to consumers.

In the context of the invention "pharmaceutical compounding" means, in contrast to compounding in pharmacies or hospital pharmacies (known as "traditional" compounding), the creation of a particular pharmaceutical product to fit the unique need of a patient to be performed in an industrial (large-scale) pharmaceutical compounding facility in compliance with the applicable drug regulatory provisions, approvals and authorizations. "Traditional" compounding, in the state of the art is most routine in the case of intravenous/parenteral medication, typically by hospital pharmacists, but is also offered by privately owned compounding pharmacies and certain retail pharmacies for various forms of medication. Whether routine or rare, intravenous or oral, etc., when a given drug product is made or modified to have characteristics that are specifically prescribed for an individual patient. However, in the context of the invention, pharmaceutical compounding is meant to denote a large-scale compounding in pharmaceutical, industrial manufacturing facility which is subject to proper regulatory control and monitoring for reason of appropriate patient safety, wherein in the said pharmaceutical compounding facility the compounding is done on bulk production of a given formulation rather than patient-specific production. It is known as "non-traditional" compounding, which is instead of "compounding" can rather be also understood as pharmaceutical "manufacturing".

Physiological diluent, aqueous physiological diluent, is referred to a as physiological solution or isotonic solution, because it closely approximates isotonic, that is, physiologically normal, solution; although neither of those names is technically accurate, because they are not exactly like blood serum, they convey the practical effect usually seen in good fluid balance with minimal hypotonicity or hypertonicity. Herein, "tonicity" is a measure of the effective osmotic pressure gradient, as defined by the water potential of two solutions separated by a semipermeable membrane. In other words, tonicity is the relative concentration of solutes dissolved in solution which determine the direction and extent of diffusion. It is commonly used when describing the response of cells immersed in an external solution. Unlike osmotic pressure, tonicity is influenced only by solutes that cannot cross the membrane, as only these exert an effective osmotic pressure. Solutes able to freely cross the membrane do not affect tonicity because they will always be in equal concentrations on both sides of the membrane. It is also a factor affecting imbibition. There are three classifications of tonicity that one solution can have relative to another: hypertonic, hypotonic, and isotonic.

A vial (also known as a phial or flacon) is a small glass or plastic vessel or bottle, often used to store medication such as liquids or powders. They are also used as scientific sample vessels. Modern vials are often made out of plastic, such as polypropylene, or sometimes glass. They are often used as storage for small quantities of liquid used in medical or molecular biology applications. There are several different types of commonly used closure systems. For glass vials, options include screw vials (e.g. closed with a screw cap), lip vials (closed with e.g. a plastic stopper) and crimp vials (e.g. closed with a rubber stopper and a metal cap).

An ampoule (also ampul, ampule, or ampulla) is a small sealed vial which is used to contain and preserve a sample, usually a solid or liquid. Ampoules are commonly made of glass, although plastic ampoules do exist. Modern ampoules are most commonly used to contain pharmaceuticals and chemicals that must be protected from air and contaminants. They are hermetically sealed by melting the thin top with an open flame, and usually opened by snapping off the neck. If properly done, this last operation creates a clean break without any extra glass shards or slivers; but the liquid or solution may be filtered for greater assurance. However, glass particle contamination may be a concern.

A syringe is a simple reciprocating pump consisting of a plunger (actually a piston) that fits tightly within a cylindrical tube (called a barrel). The plunger can be linearly pulled and pushed along the inside of the tube, allowing the syringe to take in and expel a liquid or gas through a discharge orifice at the front (open) end of the tube. The open end of the syringe may be fitted with a needle, a nozzle, or a tubing to help direct the flow into and out of the barrel. Syringes may be foreseen as a single-use syringe.

A Luer connector or Luer taper is a standardized system of small-scale fluid fittings used for making leak-free connections between a male-taper fitting and its mating female part on medical and laboratory instruments, including hypodermic syringe tips and needles or stopcocks and needles. There are two varieties of Luer taper connections: locking and slipping. Their trade names are confusingly similar to the non proprietary names. "Luer-Lok" and "Luer-slip" are registered trademarks of Becton Dickinson. "Luer-Lok" style connectors are often generically referred to as "Luer lock", and "Luer-slip" style connectors may be generically referred to as "slip tip". Luer lock fittings are securely joined by means of a tabbed hub on the female fitting which screws into threads in a sleeve on the male fitting. "Luer lock" style connectors are divided into two types "one piece luer lock" and "two piece luer lock or rotating collar luer lock". One piece Luer lock comes as a single mold, and locking is achieved by rotating the entire luer connector or system. In two piece luer lock, a free rotating collar with threads is assembled to the luer and the locking is achieved by rotating the collar. Slip tip (Luer-slip) fittings simply conform to Luer taper dimensions and are pressed together and held by friction (they have no threads). Luer components are manufactured either from metal or plastic and are available from many companies worldwide.

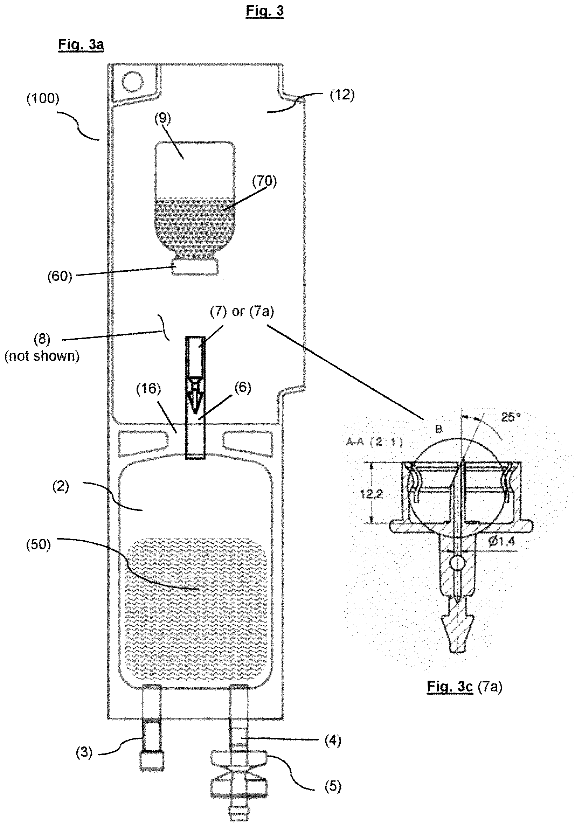

A so-called Primed Sharp connector may also be used to attach and fix the container (9), and for example a container (9s) which is a syringe, e.g. a single-use syringe. See, for example, in Fig. 3c, where a schematic Primed Sharp connector is displayed.

### Embodiments of the invention:

The instant invention is defined in claim 1. Preferred aspects of the invention are defined in the dependent claims. Embodiments herein after exemplify the invention, some of them being directed to the components of the kit of the invention as defined in said claim 1.

In particular, in an embodiment 1, the present invention pertains to a sterile and closed prefilled drug containing (medical) device system for administering, preferably for use in a treatment of administering, at least one pharmaceutically active substance (drug) by pressurized aerosol therapy (PAT) in a hollow organ, or preferably in a body cavity, of a patient, in particular a therapeutic pneumoperitoneum, comprising a flexible package (100, 101), comprising:
- at least one container (9) comprising at least one pharmaceutically active substance (70) in powder, lyophilisate, solution, liquid, gel or other form (suspension, emulsion), and being provided with breakable, an openable, or perforable cap (60); and
- a bag (2) of (pharmaceutically or physiologically acceptable) liquid diluent (50), in particular connectable to an aerosol generating means, preferably connectable to an application lance and/or injection lance with nozzle and/or a pumping system, preferably a micro-pump, for use with an application lance and/or injection lance with nozzle,
   ∘ wherein the liquid diluent (50) is preferably a physiologically acceptable aqueous diluent;
   ∘ wherein the bag (2) is optionally equipped with at least one drain tube (4) provided with a closing device (5); preferably wherein the optional at least one drain tube (4), after removal of the closing device (5), serves as connector to an aerosol generating means, preferably as connector to an application lance and/or injection lance with nozzle and/or to a pumping system, preferably to a micro-pump, for use with an application lance and/or injection lance with nozzle;
   ∘ and wherein the bag (2) is equipped with a mixing tube (6), optionally equipped with an openable closure (7), and which optionally ends with a perforation device (8), or which optionally ends with a coupling and perforation device (8), for a container (9), which container (9) is preferably a vial, syringe, single-use syringe, ampoule, phial, or bottle (9), comprising the pharmaceutically active substance (70) in powder, lyophilisate, solution, liquid, gel or other form (suspension, emulsion), and which container (9) is provided with breakable, an openable, or perforable cap (60);
   and

wherein said package (100, 101) is comprising at least one flexible airtight sterile casing (12), containing said container (9), preferably a vial, syringe, single-use syringe, ampoule, phial, or bottle (9), of the said pharmaceutically active substance, and said perforation device (8), optionally the said coupling and perforation device (8), said container (9) being housed in the casing (12), optionally in a coupling position with the said optional coupling and perforation device (8); and
wherein the said container (9) is manually maneuverable from the outside of the casing (12) in order to be opened inside the closed casing (12), optionally is manually maneuverable from the outside of the casing (12) up to a perforating position of said cap (60) through the same perforation device (8), optionally through the same coupling and perforation device (8); and
whereupon after opening of the said container (9) and of the said closure (7), and selectively mixing (i.e. dissolving, reconstituting) the at least one pharmaceutically active substance (drug) (70) with the liquid diluent (50) through the mixing tube (6), a solution of the at least one pharmaceutically active substance (drug) (70) in the liquid diluent (50) is provided for administering by pressurized aerosol therapy (PAT) in a hollow organ, or preferably in a body cavity of a patient.

Regarding the before said embodiment 1 of the present invention, a proviso may apply such that in case the at least one container (9) is a single container selected from a vial, phial or bottle, then the sterile and closed prefilled drug containing (medical) device system is solely for use in a treatment of administering at least one pharmaceutically active substance (drug) by pressurized aerosol therapy (PAT) in a hollow organ, or preferably in a body cavity, of a patient, in particular a therapeutic pneumoperitoneum.

Item (8) which may be shown or only indicated as not shown in the Figures represent a perforation device (8), optionally combined as a coupling and perforation device (8), which may be of conventional nature and applicable as known in the art, or may be constructed as described herein. Item (3) shown in the Figures may be a conventional supply tube, optionally also terminally equipped with an optionally perforable closing (5), said item (3) allowing the prefilling with the (pharmaceutically or physiologically acceptable) liquid diluent (50), and optionally, if desired and/or in case of therapeutic or other treatment need, may also be used for introducing an additional medication and/or agent and/or solvent and/or additional same or different and/or different liquid diluent (50). Item (3), after the prefilling, may also be present as perforable or dead-end closed, welded up or otherwise sealed, tube-end. Item (16) shown in the Figures represents the separating wall between the casing (12) and the bag (2) for the liquid diluent.

Regarding the optional at least one drain tube (4) it is noted that, after removal of the closing device (5), it may serve as connector to an aerosol generating means, preferably as connector to an application lance and/or injection lance with nozzle and/or to a pumping system, preferably to a micro-pump, for use with an application lance and/or injection lance with nozzle.

Prefilling according to the invention can be achieved by methods known to the person skilled in the art. For example the prefilling can be achieved in the following two ways: In the normal pharmaceutical manufacturing procedure, the drug containing (medical) device system and/or any materials and components thereof is tested for stability (stability test) for every single drug, and its distribution in a prefilled form requires a complete marketing authorization file, or a file for changes made in the drug containing (medical) device system and/or any materials and components, or packaging thereof, i.e., from material "a" to material "b", or the same material but in a different size, since drugs are generic supported by a different file. Pharmaceutical prefilling is preferably carried out under vacuum or modified atmosphere in an authorized pharmaceutical company under the supervision of the relevant authorities, observing all the necessary specifications and in a controlled-storage system for controlled substances (in a locked warehouse).

In the second way, prefilling is meant as compounding, filling in a pharmaceutical compounding facility inside an already sterilized container, preferably under vacuum or modified atmosphere conditions, wherein the test data (in specific authorized labs) show stability duration of more than one month or longer as specified herein, time considered to be necessary for storage and/or for the patient's benefits in distribution, the avoidance of unnecessary moves by the nursing staff and the destruction of expired prefilled drugs owing to the short stability duration. The present invention enables the normal operation of a pharmaceuticals warehouse, avoiding quick circulation (import-export) of drugs with short expiration times.

In the context of the invention, any pharmaceutically or physiologically acceptable solution, in particular aqueous solution, for example but not limited to, of physiologically acceptable inorganic and/or organic salts, sugars, buffers (e.g. phosphate as a buffer) and the like, and combinations thereof, may be used as the diluent. Non-limiting examples of a physiological diluent, in particular aqueous physiological diluent, are saline, also known as saline solution, i.e. normal saline; physiological glucose solution; physiological mannitol solution; Ringer's solution, Ringer's lactate solution, Tyrode's solution; and other or physiological solution known in the art.

Saline, also known as saline solution, is a mixture of sodium chloride in water and has a number of uses in medicine, including its use to dilute other medications, for example, to be given by injection or infusion. Normal saline (NSS, NS or N/S) is the commonly used phrase for a solution of 0.90% w/v of NaCl, or 9.0 g per litre. This solution is also referred to as physiological saline or isotonic saline (because it closely approximates isotonic, that is, physiologically normal, solution).

In an example of the present invention, the pharmaceutically or physiologically acceptable solution (diluent), in particular aqueous solution (diluent), may be a physiologically acceptable aqueous solution of sodium chloride (0.9 % w/v) and/or glucose (5 % w/v).

Ringer's solution is a solution of several salts dissolved in water for the purpose of creating an isotonic solution relative to the body fluids of an animal. Ringer's solution typically contains sodium chloride, potassium chloride, calcium chloride and sodium bicarbonate, with the last used to balance the pH. Other additions can include chemical fuel sources for cells, including ATP and dextrose, as well as antibiotics and antifungals. To produce a standard isotonic solution 6.5g NaCl, 0.42g KCI, 0.25g CaCl2 and 0.2g of sodium bicarbonate is dissolved in one litre of distilled water.

Ringer's lactate solution, also known as sodium lactate solution and Hartmann's solution, is a mixture of sodium chloride, sodium lactate, potassium chloride, and calcium chloride in water. It is normally used for replacing fluids and electrolytes in those who have low blood volume or low blood pressure.

Tyrode's solution is a solution that is roughly isotonic with interstitial fluid and used in physiological experiments and tissue culture. It resembles lactated Ringer's solution, but contains magnesium, a sugar (usually glucose) and uses bicarbonate and phosphate as a buffer instead of lactate. Some variations also include phosphate and sulfate ions. It must be gassed with 95% oxygen 5% carbon dioxide when used for cell culture applications and physiology experiments in order to achieve an appropriate pH.

In an embodiment 2, the present invention pertains to a sterile and closed prefilled drug containing (medical) device system for administering, preferably for use in a treatment of administering, at least one pharmaceutically active substance (drug) by pressurized aerosol therapy (PAT) in a hollow organ, or in a body cavity, of a patient, in particular a therapeutic pneumoperitoneum, comprising a flexible package (100, 101), comprising:
- at least one container (9) comprising at least one pharmaceutically active substance (70) in powder, lyophilisate, solution, liquid, gel or other form (suspension, emulsion), and being provided with breakable, an openable, or perforable cap (60); and
- a bag (2) of liquid diluent (50), in particular connectable to an aerosol generating means, preferably connectable to an application lance and/or injection lance with nozzle and/or a pumping system, preferably a micro-pump, for use with an application lance and/or injection lance with nozzle,
   ∘ wherein the liquid diluent (50) is preferably a physiologically acceptable aqueous diluent;
   ∘ wherein the bag (2) is optionally equipped with at least one drain tube (4) provided with a closing device (5); preferably wherein the optional at least one drain tube (4), after removal of the closing device (5), serves as connector to an aerosol generating means, preferably as connector to an application lance and/or injection lance with nozzle and/or to a pumping system, preferably to a micro-pump, for use with an application lance and/or injection lance with nozzle;
   ∘ and wherein the bag (2) is equipped with a mixing tube (6), optionally equipped with an openable closure (7), and which optionally ends with a perforation device (8), or which optionally ends with a coupling and perforation device (8), for a container (9), which container (9) is preferably a vial, syringe, single-use syringe, ampoule, phial, or bottle (9), comprising the pharmaceutically active substance (70) in powder, lyophilisate, solution, liquid, gel or other form (suspension, emulsion), and which container (9) is provided with breakable, an openable, or perforable cap (60);
   and

wherein said package (100, 101) is comprising at least one flexible airtight sterile casing (12), containing said container (9), preferably a vial, syringe, single-use syringe, ampoule, phial, or bottle (9), of the said pharmaceutically active substance, and said perforation device (8), optionally the said coupling and perforation device (8), said container (9) being housed in the casing (12), optionally in a coupling position with the said optional coupling and perforation device (8); and
wherein the said container (9) is manually maneuverable from the outside of the casing (12) in order to be opened inside the closed casing (12), optionally is manually maneuverable from the outside of the casing (12) up to a perforating position of said cap (60) through the same perforation device (8), optionally through the same coupling and perforation device (8); and

whereupon after opening of the said container (9) and of the said closure (7), and selectively mixing (i.e. dissolving, reconstituting) the at least one pharmaceutically active substance (drug) (70) with the liquid diluent (50) through the mixing tube (6), a solution of the at least one pharmaceutically active substance (drug) (70) in the liquid diluent (50) is provided for administering by pressurized aerosol therapy (PAT) in a hollow organ, or preferably in a body cavity of a patient;
and further or alternatively comprising at least one multiple-way valve (10) connectable to or connecting the interior space of bag (2) and the interior space of at least one flexible airtight sterile casing (12) of said package (100, 101), preferably wherein the multiple-way valve (10) is selected from the group of a three-way valve, a three-way slipper valve, a three-way cock, a three-way stopcock.

In an embodiment 3, the present invention pertains to a sterile and closed prefilled drug containing (medical) device system for administering, preferably for use in a treatment of administering, at least one pharmaceutically active substance (or drug) by pressurized aerosol therapy (PAT) in a hollow organ, or in a body cavity, of a patient, according to embodiment 1 or embodiment 2, wherein the said prefilled drug containing (medical) device system is in compliance, preferably is obtained by manufacturing is in compliance, with the guidelines of a competent drug regulatory authority applicable for pre-filled systems, preferably for pre-filled systems for long-term storage.

In an embodiment 4, the present invention pertains to a sterile and closed prefilled drug containing (medical) device system for administering, preferably for use in a treatment of administering, at least one pharmaceutically active substance (or drug) by pressurized aerosol therapy (PAT) in a hollow organ, or in a body cavity, of a patient, according to any of the preceding claims, for non-systemic (therapeutic) efficacy, preferably for local and/or topical (therapeutic) efficacy in a hollow organ, or in a body cavity, of a patient.

In an embodiment 5, the present invention pertains to a sterile and closed prefilled drug containing (medical) device system for administering, preferably for use in a treatment of administering, at least one pharmaceutically active substance (drug) by pressurized aerosol therapy (PAT) in a hollow organ, or in a body cavity, of a patient, according to embodiment 4, wherein the therapeutically effective dose for local and/or topical efficacy of the at least one pharmaceutically active substance per treatment:
(i) is the same as the therapeutically effective dose of the corresponding systemic treatment, in particular based on the maximum dose of the corresponding systemic treatment; or
(ii) is less than the therapeutically effective dose of the corresponding systemic treatment, in particular based on the maximum dose of the corresponding systemic treatment;
   and preferably
(iii) is less than 100 %, about equal or less than one of 99 %, 98 %, 97 %, 96 %, 95 %, 94 %, 93 %, 92 %, 91 %, 90 %, 85 %, 80 %, 75 %, 70 %, 65 %, 60 %, 55 %, 50 %, 45 %, 40 %, 35 %, 30 %, 25 %, 20 %, 15 %, each relative to the therapeutically effective dose of the corresponding systemic treatment, in particular based on the maximum dose of the corresponding systemic treatment;
   and more preferably,
   in particular, but not limited to, in case the pressurized aerosol therapy (PAT) is selected from pressurized intra-peritoneal aerosol chemotherapy (PIPAC),
(iv) is about equal or less than 50 %, about equal or less than 45 %, about equal or less than 40 %, about equal or less than 35 %, about equal or less than 30 %, about equal or less than 25 %, about equal or less than 20 %, about equal or less than 15 %, about equal or less than 10 %, each relative to the therapeutically effective dose of the corresponding systemic treatment, in particular based on the maximum dose of the corresponding systemic treatment; or
(v) is about 5-50 %, about 5 - 45 %, about 5-40 %, about 5 - 35 %, about 5-30 %, about 5 - 25 %, about 5 - 20 %, about 5 - 15 %, about 6 - 15 %, about 7 - 15 %, about 8 - 15 %, about 9 - 15 %, about 10 - 15 %, about 8-14 %, about 9 - 14 %, about 8 - 13 %, about 9 - 13 %, about 8 - 12 %, about 9 - 12 %, about 9 - 11 %; preferably about 10 % ± 3 %, about 10 % ± 2 %, about 10 % ± 1 %; each relative to the therapeutically effective dose of the corresponding systemic treatment, in particular based on the maximum dose of the corresponding systemic treatment.

In an embodiment 6, the present invention pertains to a sterile and closed prefilled drug containing (medical) device system for administering, preferably for use in a treatment of administering, at least one pharmaceutically active substance (drug) by pressurized aerosol therapy (PAT) in a hollow organ, or in a body cavity, of a patient, according to any of the preceding embodiments, wherein the patient is a mammalian patient, preferably a human patient.

In an embodiment 7, the present invention pertains to a sterile and closed prefilled drug containing (medical) device system for administering, preferably for use in a treatment of administering, at least one pharmaceutically active substance (drug) by pressurized aerosol therapy (PAT) in a hollow organ, or preferably in a body cavity, of a patient, according to any of the preceding embodiments,
wherein as a hollow space the body cavity is selected from the group consisting of each hollow space in the body that is lined by mesothelium and epithelium, self-contained or connected with other cavities or the outside world, such as the abdomen, peritoneal cavity, thoracic cavity, luminal cavity, vesical cavity, cranial cavity, nasal cavity, oral cavity, pharynx, subarachnoid spaces, or joint cavities; preferably wherein the body cavity is selected from the group consisting of the peritoneal cavity (peritoneum), thoracic cavity (thorax), luminal cavity (lumen), vesical cavity (vesicle), uterus;
or wherein as hollow space the hollow organ is selected from the group consisting of such as the cavity of the heart, blood vessels, bile duct, urinary tract, gastrointestinal tract, uterus or brain ventricles.

In an embodiment 8, the present invention pertains to a sterile and closed prefilled drug containing (medical) device system for administering, preferably for use in a treatment of administering, at least one pharmaceutically active substance (drug) by pressurized aerosol therapy (PAT) is in a body cavity of a patient, according to any of the preceding embodiments, wherein the pressurized aerosol therapy (PAT) is selected from the group consisting of pressurized intra-peritoneal aerosol chemotherapy (PIPAC), pressurized intra-thoracic aerosol chemotherapy (PITAC), pressurized intra-luminal aerosol chemotherapy (PILAC), and pressurized intra-vesical aerosol chemotherapy (PIVAC), pressurized intra-rectal aerosol chemotherapy (PIRAC).

PIPAC is technically as simple as a diagnostic laparoscopy. The treatment occurs minimally invasive. By using two small incisions (cuts) in the abdominal wall, the medication - under pressure, by means of a laparoscopic nebulizer - is blown as a fine mist into the abdominal cavity, where it is pressed into the diseased tissue. Thereby it has a substantially better effect on the tumor. This result comes up with only 10 percent of the usual dose of medication. The side effects are very small, therefore the therapy is very well tolerated by the patients. PIPAC aims for an extension of life by means of the control of symptoms. PIPAC's first results demonstrate both: PIPAC is associated with fewer side effects than traditional therapies and furthermore the performance index increases with the therapy. The first data regarding the chances of survival are encouraging.

Ovarian cancer (ovarian carcinoma) and metastases in the peritoneum: Ovarian cancer (ovarian carcinoma) is the second most common malignant disease of females' genital organs. In a current study of the Marien Hospital Herne (University Hospital of the Ruhr-University Bochum), under the direction of Prof. Dr. Clemens Tempfer (chief physician of the clinic of gynecology and obstetrics), the use of compression-aerosol chemotherapy in patients with recurrent ovarian cancer and metastases in the peritoneum was examined. First results are encouraging. During the so-called PIPAC therapy (Pressurized Intra Peritoneal Aerosol Chemotherapy) chemotherapy agents are specifically introduced into the abdominal cavity. This happens in order to directly reach and push back the cancerous tumors. This new way with already approved medication, was chosen for the therapy against ovarian and peritoneal cancer, as these agents immediately penetrate into the diseased tissue.

PITAC (Pressurized IntraThoracic Aerosol Chemotherapy): The pressure aerosol chemotherapy can also be used in the chest, for example regarding malignant pleural effusion or regarding pleural mesothelioma. PITAC is a further preferred aspect of the present invention as it described herein. PITAC is technically as simple as a diagnostic thoracoscopy.

PILAC (Pressurized Intraluminal Aerosol Chemotherapy): In the treatment of PILAC the pressure aerosol chemotherapy is applied with a mirroring between two balloons into the esophagus. As in the treatment of PIVAC this innovative method only has been applied in the animal. Besides the technical feasibility has been demonstrated, and the penetration depth of a drug (small-molecule drug) into the muscular wall of the esophagus was documented.

PIVAC (Pressurized IntraVesical Aerosol Chemotherapy): In matters of PIVAC the pressure aerosol chemotherapy is administered into the bladder. Up to now, this innovative method was only used in animal testing, and the technical feasibility was demonstrated.

PIRAC (Pressurized IntraRectal Aerosol Chemotherapy): The pressure aerosol chemotherapy can also be used via the rectum, for example regarding malignant cancer of the lower intestinal tract, e.g., the colon and/or the small intestine.

In an embodiment 9, the present invention pertains to a sterile and closed prefilled drug containing (medical) device system for administering, preferably for use in a treatment of administering, at least one pharmaceutically active substance (drug) by pressurized aerosol therapy (PAT) in a hollow organ, or in a body cavity, of a patient, according to any of the preceding embodiments, wherein the at least one pharmaceutically active substance (drug) is selected from
(a) the group consisting of antitumor, anticancer, cytostatic, antinecrotic, antimicrobial, antimycotic, antibacterial, antiviral, antiseptic, antibiotic substances or a combination thereof; preferably anticancer, antitumor or cytostatic substances, or a combination thereof; and/or
(b) the group consisting of anticancer, antitumor, cytostatic substances or a combination thereof, preferably selected from the group consisting of
   aflibercept, amsacrin, alemtuzumab, arsene trioxide, asparaginase, azacitidine, bendamustin hydrochloride, bevacizumab, bleomycin, blinatumomab, bortezomib, busulfan, cabacitaxel, carboplatin, carfilzomib, carmustine, 2 CdA /cladribin, cetuximab, cidofovir, cisplatin, clofarabin, cyclophosphamide, cytarabin, dacarbacin, dactinomycin, daratumab, daunorubicin, decitabin, docetaxel, doxorubicin, liposomales doxorubicin, eribulin mesilate, epirubicin, etoposid, fludarabin, 5-flourouracil, foscarnet, gemicitabin, glancyclovir, idarubicin, ifosfamide, ipilmumab, irinotecan, melphalan, methotrexat, mifamurtid, mitomycin, mitoxantron, nelarabin, nivolumab, obinituzumab ofatumumab, oxaliplatin, paclitaxel, panitumab, pemetrexed, pertuzumab, prembolizumab, ramucirumab, rituximab, romidepsin, thiotepa, topotecan, trabectedin, trastuzumab, trastuzumab-emtansin, treosulfan, vinblastine, vincristin, vindesin, vinflunin, vinorelbin,
   more preferably selected from the group consisting of doxorubicin, cisplatin, oxaliplatin, topotecan, paclitaxel, pemetrexed, bendamustin, carboplatin, irinotecan, docetaxel, mitomycin, epirubicin, vinorelbin, or a combination thereof;
   preferably is selected from one of:
(c) a combination of doxorubicin, cisplatin, oxaliplatin, topotecan, and paclitaxel;
(d) a combination of pemetrexed, bendamustin, topotecan, and carboplatin;
(e) a combination of irinotecan and docetaxel;
(f) a combination of doxorubicin, mitomycin, and epirubicin;
(g) a combination of docetaxel, oxaliplatin, paclitaxel, and vinorelbin.

As an example, the said drug combinations may be used in dosages as exemplified in Table I (based on body surface area "BSA"):

**Table I: Dosages of Drug Combination**

| Table Ia: Dosage PIPAC (IntraPeritoneal) | | | | | | |
|---|---|---|---|---|---|---|
| | | Doxorubicin [mg] | Cisplatin [mg] | Topotecan [mg] | Oxaliplatin [mg] | Paclitaxel [mg] |
| For very small, petite women the dosage is usually: | | | | | | |
| BSA | 1.25 | 1.88 | 9.38 | 0.50 | 16.25 | 25.00 |
| For very tall, heavy men the dosage is usually: | | | | | | |
| BSA | 3.00 | 4.50 | 22.50 | 1.20 | 39.00 | 60.00 |
| For assumption of a constant dosage, the dosage is usually: | | | | | | |
| BSA | 2.00 | 3.00 | 15.00 | 0.80 | 26.00 | 40.00 |

| Table Ib: Dosage PITAC (IntraThoracic) | | | | | | |
|---|---|---|---|---|---|---|
| | | Pemetrexed [mg] | Bendamustin [mg] | Topotecan [mg] | Carboplatin [mg] | |
| For very small, petite women the dosage is usually: | | | | | | |
| BSA | 1.25 | 62.50 | 12.50 | 0.50 | 50.00 | |
| For very tall, heavy men the dosage is usually: | | | | | | |
| BSA | 3.00 | 150.00 | 30.00 | 1.20 | 120.00 | |
| For assumption of a constant dosage, the dosage is usually: | | | | | | |
| BSA | 2.00 | 100.00 | 20.00 | 0.80 | 80.00 | |

| Table Ic: Dosage PILAC (IntraLuminal) | | | | | | |
|---|---|---|---|---|---|---|
| | | Irinotecan [mg] | Docetaxel [mg] | | | |
| For very small, petite women the dosage is usually: | | | | | | |
| BSA | 1.25 | 43.75 | 12.50 | | | |
| For very tall, heavy men the dosage is usually: | | | | | | |
| BSA | 3.00 | 105.00 | 30.00 | | | |
| For assumption of a constant dosage, the dosage is usually: | | | | | | |
| BSA | 2.00 | 70.00 | 20.00 | | | |

| Table Id: Dosage PIVAC (IntraVessical) | | | | | | |
|---|---|---|---|---|---|---|
| | | Doxorubicin [mg] | Mitomycin [mg] | Epirubicin [mg] | | |
| For very small, petite women the dosage is usually: | | | | | | |
| BSA | 1.25 | 1.88 | | | | |
| For very tall, heavy men the dosage is usually: | | | | | | |
| BSA | 3.00 | 4.50 | | | | |
| For assumption of a constant dosage, the dosage is usually: | | | | | | |
| BSA | 2.00 | 3.00 | 8.00 | 10.00 | | |

| Table le: Dosage PIRAC (IntraRectal) | | | | | | |
|---|---|---|---|---|---|---|
| | | Docetaxel [mg] | Vinorelbin [mg] | Oxaliplatin [mg] | Paclitaxel [mg] | |
| For very small, petite women the dosage is usually: | | | | | | |
| BSA | 1.25 | 12.50 | 3.75 | 16.25 | 25.00 | |
| For very tall, heavy men the dosage is usually: | | | | | | |
| BSA | 3.00 | 30.00 | 9.00 | 39.00 | 60.00 | |
| For assumption of a constant dosage, the dosage is usually: | | | | | | |
| BSA | 2.00 | 20.00 | 6.00 | 26.00 | 40.00 | |

Typical standard and maximum dosages of cytostactic compounds may be exemplified as indicated in the following Table II.

**Table II: Listing of Typical Standard (normal) and Maximum Dosages of Cytotsatic Compounds (conventional treatments).**

| **Substance** | **Standard Dosage** | **Maximum Dosage** |
|---|---|---|
| Paclitaxel human serum albumin bonded nanoparticles | 125 mg/m2 (adenocarcinoma of the pancreas) | 275 mg |
| | 260 mg/m2 | 570 mg |
| | (Breast cancer) | |
| Doxorubicin | 60-75 mg/m2 | 120 mg 1 x every 2 weeks |
| | | 150 mg 1 x every 3 weeks |
| | | Cumulative anthracyclines: maximum 450 mg/m2 |
| Pemetrexed | 500 mg/m2 | 1100 mg every 3 weeks |
| Melphalan | 8-30 mg/m2 | 36 mg / intravesikulär 100 mg |
| Amsacrin | 120 mg/m2 | hematology up to 300 mg |
| Ofatumumab | 300 mg (1st dose) | 300 mg (1st dose) |
| | 2000 mg (following dose) | 2000 mg (following dose) |
| Asparaginase | hematology: 10'000 IU/m2 | 20'000 IU/m2 |
| Nelarabin | 1500 mg/m2 (3 x per cycle) | 3500 mg/m2 |
| Bevacizumab | 5 mg/kg, resp. 10 mg/kg | 5/7.5/10/15 mg/kg |
| | every 2 weeks | |
| | resp. 15 mg/kg | |
| | every 3 weeks | |
| Bleomycin | | Over 80 years: |
| | | 15,000 IU 15 mg / week |
| | | Cumulative: |
| | | 100'000 IU = 100 mg |
| | | 70-79 |
| | | years: 30'000 IU = 30 mg / week |
| | | Cumulative: |
| | | 150'000 - 200'000 = 150 - 200 mg |
| | | 60 - 69 years: |
| | | 30'000 IU - 60'000 IU = 30 -60 mg / week |
| | | Cumulative: |
| | | 200'000 - 300'000 IU = 200-300 mg |
| | | Less than 60 years: |
| | | 30'000 IU - 60'000 IU = 30 -60 mg / week |
| | | Cumulative: |
| | | 400'000 IU = 400 mg |
| | | (In pleurodesis 60,000 IU 60 mg) |
| | | Cumulatively maximum 270 mg |
| | | (Corresponding to 3 x BEP) |
| Blinatumomab | 9 µg / day | |
| | 1 cycle 1st week | |
| | 28 µg / day the following administrations | |
| Liposomal Doxorubicin | 0.8 mg/kg/dose 4 x daily or | Total dose per cycle 0.8 mg/kg/6-h. |
| | 3.2 mg/kg as single dose 1x daily over 2-4 days | 3,2 mg/kg/d = 4 x 0.8 mg/kg/d (total 16 single doses) |
| Alemtuzumab | | 30 mg 3 x per week |
| Carboplatin | 200-400 mg/m2 (AUC 2, 4-7) | 1500 mg (AUC 7) |
| | | 1300 mg (AUC 6) |
| | | 1 x every 3 weeks |
| | | 1100 mg (AUC 5) |
| | | 1 x every 3 weeks |
| | | 900 mg (AUC 4) |
| | | 1 x every 3 weeks |
| | | 450 mg (AUC 2) weekly |
| Carmustine | 80-200 mg/m2 | 600 mg once only |
| | 300 mg/m2 | |
| Cisplatin | 15-100 mg/m2 | 220 mg |
| | 40 mg/m2 weekly | 1 x every 3 weeks |
| Dactinomycin | 0.4-0.6 mg/m2 | 1 mg daily during 5 days, every 3 weeks |
| Gancyclovir | Initial and maintenance dose according to requirements of compendium | Creatinine Clearance (ml/min/1.73m2) |
| | | > 50 5.0 mg/kg - 12h |
| | | 25-50 2.5 mg/kg - 12h |
| | | 10-25 2.5 mg/kg - 24h |
| | | 0-10 1.25 mg/kg - 24h |
| Ramucirumab | 8 mg/kg | 8 mg/kg |
| Cytarabin | 100-2000 mg/m² at Hyper-CVAD | 4200 mg at Hyper-CVAD |
| | 3000 mg/m² | 6300 mg |
| | 4 x every 12h | 4 x every 12h |
| Dacarbazin | 150-800 mg/m2 | 1700 mg every 3 weeks |
| Decitabin | 20 mg/m2 on 5 subsequent days, every 4 weeks | |
| Daratumumab | 16 mg/kg | 16 mg/kg |
| Daunorubicin | 40-60 mg/m2 | 120 mg |
| Cytarabin | 50 mg intrathekal every 2 weeks | 50 mg intrathekal every 2 weeks |
| Vindesin | 3-4 mg/m2 | 6 mg per week |
| Oxaliplatin | 130 mg/m2 | 280 mg per 3weeks |
| Cyclophosphamid | 80-1600 mg/m² | 3300 mg |
| Cetuximab | first dose 400 mg/m2, then 250 mg/m2/week | 900 mg first dose otherwise 600 mg |
| | 500 mg/m2 every 2 weeks | every 2 weeks 1200 mg |
| Etoposid (VP-16) | 60-125 mg/m2 150 mg/m2 (VIDE) | 280 mg daily at 3 days every 3 weeks |
| | for testicular cancer (BEP-scheme): 100 mg/m2 daily over 5 days | for testicular cancer (BEP-scheme): 250 mg daily over 5 days |
| Clofarabin | 30 mg/m2 | 50 mg/m2 |
| Epirubicin | 60-120 mg/m2 | 240 mg every 3 weeks |
| | | cumulative anthracycline: 900 mg/m2 |
| Fludarabin | 25 mg/m2 | |
| 5-Fluorouracil | 375-500 mg/m2 (bolus) 1 x per week 1000 mg/m2/24 h | 1200 mg (bolus) 1 x per week 2600 mg/24h |
| | continuous infusion over 4 to 5 days 2300 mg/m2/24 h | continuous infusion over 4 to 5 days 6000 mg/24 h |
| | continuous infusion over 24h 1x per week 2800 mg/m2/48 h | continuous infusion over 24h 1x per week 7300 mg/48 h |
| | FOLFIRI 2000 mg/m2/48 h FOLFOX | continuous infusion every 2 weeks (FOLFIRI, FOLFOX) |
| Foscarnet | 60 mg/kg 8 each hour or 90 mg/kg 12 each hou. | |
| Obinituzumab | 1000 mg | 1000 mg |
| | | day 1, 8, 15 (cycle 1) the every 28 days |
| Gemcitabin | 1250 mg/m2 | 2600 mg 1 x per week |
| Eribulin mesilate | 1,4 mg/m2 | 3 mg day 1 and 8 of a 3-weeks cycle |
| Trastuzumab | 8 mg once, then 6 mg/kg every 3 weeks resp. | 2/ 4/ 6/ 8 mg/kg |
| | 4 mg/kg every 2 weeks resp. 2 mg/kg weekly | |
| Ifosfamid | 800-5000 mg/m2 | 10 g as single dose 6 g per day at daily administration over 3-5 days |
| Topotecan | 1.25-1.5 mg/m2 /d over 5 days gynecology 4 mg/m2 weekly | 2.5 mg/d at max. 5 consecutive days |
| | | gynecology 8 mg day 1, 8, 15 in 28-days cycle |
| Irinotecan / CPT-11 | 300-350 mg/m2 | 700 mg every 3 weeks |
| Romidepsin | 14 mg/m2 | |
| Vinflunin | 320 mg/m2 | 700 mg every 3 weeks |
| Cabacitaxel | 25 mg/m2 | 55 mg every 3 weeks |
| Trastuzumab-Emtansin | 3,6 mg/kg every 3 weeks | 3.6 mg/kg |
| Pembrolizumab | 2 mg/kg every 3 weeks | 2 mg/kg |
| Carfilzomib | 20 mg/m2 first cycle or day 1+2 | |
| | 27 mg/m2 from second cycle or | |
| | 56 mg/m2 from day 8 | |
| 2 CdA / Cladribin | 0.1 -0.25 mg/kg | |
| Rituximab | 375 mg/m2 | 1050 mg |
| Mifamurtid | 2 mg/m2 | |
| Methotrexat | 40-50 mg/m2 with leucovorin to 12'000 mg/m2 | 100 mg with leucovorin to 24 g 1 x per week |
| Mitomycin | 10-15 mg/m2 | 30 mg 1 x every 6 weeks |
| Mitoxantron | 8-14 mg/m2 | 28 mg 1 x every 3 weeks |
| Nivolumab | 3 mg/kg | 3 mg/kg |
| Treosulfan | 8 g/m2 (every 3 weeks) | |
| Pertuzumab | 840 mg (1. Dosis) | 840 mg (1. Dosis) |
| | 420 mg (subsequent doses) | 420 mg (subsequent doses) |
| Bendamustin hydrochloride | 80-100 mg/m2 | 240 mg at 2 subsequent days every 3-4 weeks |
| Paclitaxel (Taxol) | 135-200 mg/m2 | 400 mg |
| | 80-100 mg/m2 weekly wöchentlich | 1 x every 3 weeks 200 mg weekly |
| Docetaxel | 75-100 mg/m2 | 220 mg 1 x every 3 weeks |
| Thiotepa | 0.3-5.0 mg/kg | 400 mg |
| Arsene trioxide | 0,15 mg/kg/day | 15 mg |
| Panitumumab | 6 mg/kg | 6 mg/kg every 2 weeks |
| Vinblastin | 4 - 6 mg/m2 | 13 mg 1x per week |
| Bortezomib | 1,3 mg/m2 day 1, 4, 8, 11 every | 2,7 mg per dose |
| | 3 weeks | 3,2 mg per dose |
| | 1,6 mg/m2 day 1, 8, 15, 22 | |
| | every 5 weeks | |
| Azacitidin | 100 mg/m2 per day | 200 mg per day |
| Vincristin | 1-1.4 mg/m2 | 2 mg every 2 weeks |
| Vinorelbin | 25-30 mg/m2 | 70 mg |
| Cidofovir | | 3 - 5 mg/kg 1 x per 1 or 2 weeks (depending on serum creatinine) |
| Ipilimumab | 3 mg/kg | 3 mg/kg every 3 weeks for in total 4 doses |
| Trabectedin | 1,5 mg/m2 | 3,3 mg |
| Aflibercept | 4 mg/kg (in combination with FOLFIRI) | 4 mg/kg |
| Idarubicin | 8-12 mg/m2 | 25 mg |

In an embodiment 10, the present invention pertains to a sterile and closed prefilled drug containing (medical) device system for administering at least one pharmaceutically active substance (drug) by pressurized aerosol therapy (PAT) in a hollow organ, or in a body cavity, of a patient, according to any of the preceding embodiments, wherein the at least one container (9) is a vial, syringe, single-use syringe, ampoule, phial, or bottle, preferably a vial, syringe, or single-use syringe.

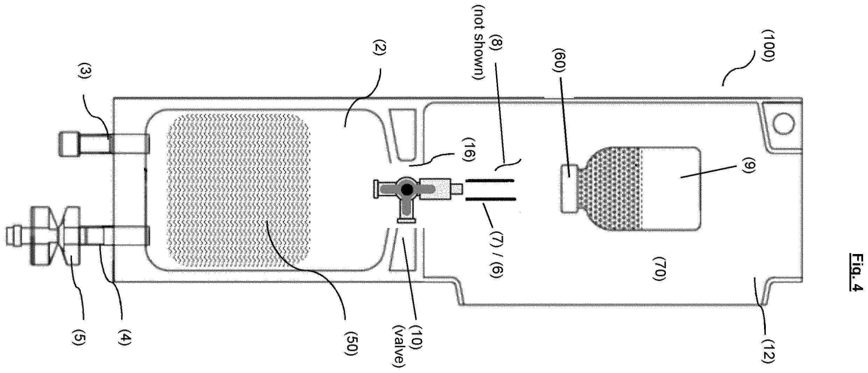

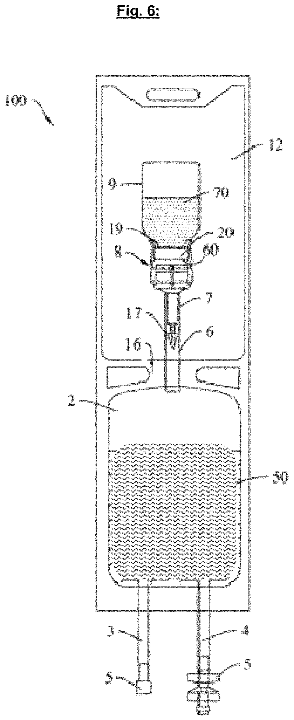

In an embodiment 11, the present invention pertains to a sterile and closed prefilled drug containing (medical) device system for administering, preferably for use in a treatment of administering, at least one pharmaceutically active substance (drug) by pressurized aerosol therapy (PAT) in a hollow organ, or in a body cavity, of a patient, according to any of the preceding embodiments, for combination therapy is comprising in the at least a flexible airtight sterile casing (12), more than one of container (9), preferably two to three containers (9a, 9b, 9c), more preferably two containers (9a, 9b), each container comprising independently at least one pharmaceutically active substance (drug). The container may particularly also be a syringe, or especially single-use syringe, (9s), which is displayed as an alternative example to the bottle (9) displayed for illustration. See Fig. 3 and 4, and in particular Fig. 5 wherein a syringe or especially single-use syringe, (9s) is displayed; optionally also in combination with a so-called Primed Sharp connector.

In an embodiment 12, the present invention pertains to a sterile and closed prefilled drug containing (medical) device system for administering, preferably for use in a treatment of administering, at least one pharmaceutically active substance (drug) by pressurized aerosol therapy (PAT) in a hollow organ, or in a body cavity, of a patient, according to any of the embodiments 1 to 10, wherein said package (100, 101) is comprising more than one of flexible airtight sterile casings (12) separated from each other, and each of the said casings is comprising independently at least one container (9), and each container is comprising independently at least one pharmaceutically active substance (drug); preferably two to three separate casings (12a, 12b, 12c), and each of the said casings (12a, 12b, 12c) is comprising independently one of the said containers (9a, 9b, 9c); more preferably two separate casings (12a, 12b), each of the said casings (12a, 12b) is comprising independently one of the said containers (9a, 9b), each container comprising independently at least one pharmaceutically active substance (drug).

In an embodiment 13, the present invention pertains to a sterile and closed prefilled drug containing (medical) device system for administering, preferably for use in a treatment of administering, at least one pharmaceutically active substance (drug) by pressurized aerosol therapy (PAT) in a hollow organ, or in a body cavity, of a patient, according to any of the embodiments 1, or 3 to 10, wherein the package (100, 101), is further characterized in that said coupling and perforation device (8) comprises a first element (21) slidably coupled with a second element (22) and movable between said coupling position of the bottle (9) and said perforating position of the cap (60) of the bottle (9), in which said first element (21) comprises a ring (23) from which at least two flaps (24) equipped with notches (25, 26) suitable to accommodate the bottle (9) in the coupling position, vertically branch off, and in that said openable closure (7) is surmounted by a circular base (28) from which pairs of further flaps (27), in turn equipped with notches (52) suitable to accommodate the bottle (9) in the perforating position of the cap (60), perimetrally and vertically side by sidebranch off, said second element (22) having gaps (30) between said couples of further flaps (27) in correspondence of said at least two flaps (24), said gaps (30) being suitable to accommodate the at least two flaps (24) with the bottle (9) in the perforating position of the cap (60).

In an embodiment 14, the present invention pertains to a sterile and closed prefilled drug containing (medical) device system for administering, preferably for use in a treatment of administering, at least one pharmaceutically active substance (drug) by pressurized aerosol therapy (PAT) in a hollow organ, or in a body cavity, of a patient, according to embodiment 13, wherein the package (100, 101) is further characterized in that
said ring (23) is inserted externally to said further flaps (27) and is configured to slide coaxially with respect to the circular base (28), and that said further flaps (27) have a curvature at their free ends such as to constitute a first limit to said first element (21) in correspondence of the bottle (9) in the coupling position, and said notches (26) are configured to contrast with said base circular (28) and form a second limit to said first element (21) in correspondence of the bottle (9) in the position of perforation of the cap (60).

In an embodiment 15, the present invention pertains to a sterile and closed prefilled drug containing (medical) device system for administering, preferably for use in a treatment of administering, at least one pharmaceutically active substance (drug) by pressurized aerosol therapy (PAT) in a hollow organ, or in a body cavity, of a patient, according to embodiment 13 or embodiment 14, wherein the package (100, 101) is further characterized in that
said further flaps (27) of the second element (22) comprise externally locking notches (53) suitable to lock the first element (21) in coupling position of the bottle (9).

In an embodiment 16, the present invention pertains to a sterile and closed prefilled drug containing (medical) device system for administering, preferably for use in a treatment of administering, at least one pharmaceutically active substance (drug) by pressurized aerosol therapy (PAT) in a hollow organ, or in a body cavity, of a patient, according to any of the embodiments 13 to 15, wherein the package (100, 101) is further characterized in that
said openable closure (7) of the coupling and perforation device (8) has internally a channel (57) ending at the top with a hollow tip (18) and inferiorly with an initial portion (17) which is frangible to allow passage of the pharmacological substance (70) in powder or another from the bottle (9), through the mixing tube (6) towards the bag (2) of liquid diluent (50).

In an embodiment 17, the present invention pertains to a sterile and closed prefilled drug containing (medical) device system for administering, preferably for use in a treatment of administering, at least one pharmaceutically active substance (drug) by pressurized aerosol therapy (PAT) in a hollow organ, or in a body cavity, of a patient, according to any of the embodiments 13 to 16, wherein the package (100, 101) is further characterized in that
said casing (12) is arranged in a position aligned with said bag (2) of liquid diluent (50) so as to be hermetically separated by a single wall (16), said mixing tube (6) being configured to extend through said single wall (16) for the communication between said bottle (9) and said bag (2) in order to introduce inside the bag (2) the pharmacological or nutritional substance (70) in powder or other contained within the bottle (9).

In an embodiment 18, the present invention pertains to a sterile and closed prefilled drug containing (medical) device system for administering, preferably for use in a treatment of administering, at least one pharmaceutically active substance (drug) by pressurized aerosol therapy (PAT) in a hollow organ, or in a body cavity, of a patient, according to any of the embodiments 13 to 17, wherein the package (100, 101) is further characterized in that
said bag (2) of liquid diluent (50) is provided with a supply tube (3) terminating with a closing device (5).

In an embodiment 19, the present invention pertains to a sterile and closed prefilled drug containing (medical) device system for administering, preferably for use in a treatment of administering, at least one pharmaceutically active substance (drug) by pressurized aerosol therapy (PAT) in a hollow organ, or in a body cavity, of a patient, according to any of the embodiments 13 to 18, wherein the package (100, 101) is further characterized in that
said casing (12) comprises internally said bag (2) of liquid diluent (50) with said drain tube (4) and said mixing tube (6) equipped with the coupling and perforation device (8) and the bottle (9).

In a particular embodiment 20, the present invention pertains to a kit, preferably a single-use kit, for administering, preferably for use in a treatment of administering, at least one pharmaceutically active substance (or drug) by pressurized aerosol therapy (PAT) in a hollow organ, or in a body cavity, in particular a therapeutic pneumoperitoneum, of a patient, comprising
(a) a sterile and closed prefilled drug containing (medical) device system for administering, preferably for use in administering at least one pharmaceutically active substance (drug) by pressurized aerosol therapy (PAT) in a hollow organ, or in a body cavity, of a patient, comprising a flexible package (100, 101) as defined in any of the embodiments 1 to 19,
   but wherein the proviso of embodiment 1 does not apply;
   and
(b) an application lance and/or injection lance with nozzle, preferably a single use (or disposable) application lance and/or injection lance with nozzle, for applying, i.e. for use in administering, the at least one pharmaceutically active substance (drug) by pressurized aerosol therapy (PAT) in a hollow organ, or in a body cavity, of a patient;
   and/or
(c) a pumping system, preferably a micro-pump, for use with an application lance and/or injection lance with nozzle for applying, i.e. for use in administering, the at least one pharmaceutically active substance (drug) by pressurized aerosol therapy (PAT) in a hollow organ, or in a body cavity, of a patient;
   and optionally
(d) (conventional) tubes and/or (conventional) connectors, for use with the .(a) the sterile and closed prefilled drug containing (medical) device system, (b) the application lance and/or injection lance with nozzle, and/or (c) the pumping system, preferably a micro-pump.

The before note on embodiment 20, of the present invention that "the proviso of embodiment 1 does not apply" means that under (a) the sterile and closed prefilled drug containing (medical) device system for administering, preferably for use in a treatment of administering, at least one pharmaceutically active substance (drug) by pressurized aerosol therapy (PAT) in a hollow organ, or preferably in a body cavity, of a patient, in particular a therapeutic pneumoperitoneum, is defined as comprising a flexible package (100, 101), comprising:
- at least one container (9) comprising at least one pharmaceutically active substance (70) in powder, lyophilisate, solution, liquid, gel or other form (suspension, emulsion), and being provided with breakable, an openable, or perforable cap (60); and
- a bag (2) of (pharmaceutically or physiologically acceptable) liquid diluent (50), in particular connectable to an aerosol generating means, preferably connectable to an application lance and/or injection lance with nozzle and/or a pumping system, preferably a micro-pump, for use with an application lance and/or injection lance with nozzle,
   ∘ wherein the liquid diluent (50) is preferably a physiologically acceptable aqueous diluent;
   ∘ wherein the bag (2) is optionally equipped with at least one drain tube (4) provided with a closing device (5); preferably wherein the optional at least one drain tube (4), after removal of the closing device (5), serves as connector to an aerosol generating means, preferably as connector to an application lance and/or injection lance with nozzle and/or to a pumping system, preferably to a micro-pump, for use with an application lance and/or injection lance with nozzle;
   ∘ and wherein the bag (2) is equipped with a mixing tube (6), optionally equipped with an openable closure (7), and which optionally ends with a perforation device (8), or which optionally ends with a coupling and perforation device (8), for a container (9), which container (9) is preferably a vial, syringe, single-use syringe, ampoule, phial, or bottle (9), comprising the pharmaceutically active substance (70) in powder, lyophilisate, solution, liquid, gel or other form (suspension, emulsion), and which container (9) is provided with breakable, an openable, or perforable cap (60);
   and

wherein said package (100, 101) is comprising at least one flexible airtight sterile casing (12), containing said container (9), preferably a vial, syringe, single-use syringe, ampoule, phial, or bottle (9), of the said pharmaceutically active substance, and said perforation device (8), optionally the said coupling and perforation device (8), said container (9) being housed in the casing (12), optionally in a coupling position with the said optional coupling and perforation device (8); and
wherein the said container (9) is manually maneuverable from the outside of the casing (12) in order to be opened inside the closed casing (12), optionally is manually maneuverable from the outside of the casing (12) up to a perforating position of said cap (60) through the same perforation device (8), optionally through the same coupling and perforation device (8); and
whereupon after opening of the said container (9) and of the said closure (7), and selectively mixing (i.e. dissolving, reconstituting) the at least one pharmaceutically active substance (drug) (70) with the liquid diluent (50) through the mixing tube (6), a solution of the at least one pharmaceutically active substance (drug) (70) in the liquid diluent (50) is provided for administering by pressurized aerosol therapy (PAT) in a hollow organ, or preferably in a body cavity of a patient.

In another particular embodiment 21, the present invention pertains to a kit, preferably a single use kit, as defined in embodiment 20 or a sterile and closed prefilled drug containing (medical) device system comprising a flexible package (100, 101) as defined in any of the embodiments 1 to 19, for use in a treatment of administering at least one pharmaceutically active substance (drug) by pressurized aerosol therapy (PAT) in a hollow organ, or in a body cavity, in particular a therapeutic pneumoperitoneum, of a patient.

In another particular embodiment 22, the present invention pertains to the a kit as defined in embodiment 20 or a sterile and closed prefilled drug containing (medical) device system comprising a flexible package (100, 101) as defined in any of the embodiments 1 to 19, for use in a treatment of cancer and/or tumor treatable by rinsing and/or perfusion, preferably in a treatment of cancer and/or tumor, including but not limited to as well as metastatic forms thereof, selected from the group consisting of cancer/tumor of the peritoneum, cancer/tumor of the urinary tract, in particular bladder cancer, ovarian cancer, cancer/tumor of the colorectum, and most preferably of cancer/tumor of the peritoneum; and/or in a treatment of cancer and/or tumor selected from the group consisting of in particular colorectal cancer, cervical cancer, uterine cancer, bladder cancer, lung cancer, esophageal cancer, gastric cancer, pancreatic cancer, peritoneal cancer, and prostate cancer, as well as metastatic forms thereof.

In a further particular embodiment 23, there is disclosed, but not claimed a method of treatment, wherein a kit as defined in embodiment 20 is used or a sterile and closed prefilled drug containing (medical) device system comprising a flexible package (100, 101) as defined in any of the embodiments 1 to 19 is used, in a treatment of administering at least one pharmaceutically active substance (drug) by pressurized aerosol therapy (PAT) in a hollow organ, or in a body cavity, in particular a therapeutic pneumoperitoneum, of a patient.

In a still further particular embodiment 24, there is disclosed, but not claimed a method of treatment according to embodiment 23, wherein a cancer and/or tumor is treated that is treatable by rinsing and/or perfusion, preferably wherein a cancer and/or tumor, including but not limited to as well as metastatic forms thereof, is treated selected from the group consisting of cancer/tumor of the peritoneum, cancer/tumor of the urinary tract, in particular bladder cancer, ovarian cancer, cancer/tumor of the colorectum, and most preferably of cancer/tumor of the peritoneum; and/or colorectal cancer, cervical cancer, uterine cancer, bladder cancer, lung cancer, esophageal cancer, gastric cancer, pancreatic cancer, peritoneal cancer, and prostate cancer, as well as metastatic forms thereof.

In a yet further particular embodiment 25, the present invention pertains to a syringe (or use thereof), especially a single-use syringe, as a container (9) comprising at least one pharmaceutically active substance (drug) (70) in powder, lyophilisate, solution, liquid, gel or other form, preferably in solution, liquid, gel form, in a sterile and closed prefilled drug containing (medical) device system, preferably as defined above, and in any of the claims 1 to 9, or for use in a kit comprising (a) a sterile and closed prefilled drug containing (medical) device system and (b) an application lance and/or injection lance with nozzle, preferably in a kit, preferably for use in a treatment of administering at least one pharmaceutically active substance (drug) (70) by pressurized aerosol therapy (PAT) in a hollow organ, or preferably in a body cavity, of a patient, in particular a therapeutic pneumoperitoneum.

In a yet another particular embodiment 26, the present invention pertains to a syringe, especially a single-use syringe, as a container (9) comprising at least one pharmaceutically active substance (drug) (70) in powder, lyophilisate, solution, liquid, gel or other form, preferably in solution, liquid, gel form, for use in the preparation of a sterile and closed prefilled drug containing (medical) device system, preferably as defined above, or in the preparation of a kit comprising (a) a sterile and closed prefilled drug containing (medical) device system and (b) an application lance and/or injection lance with nozzle, preferably a kit, preferably for use in a treatment of administering at least one pharmaceutically active substance (drug) (70) by pressurized aerosol therapy (PAT) in a hollow organ, or preferably in a body cavity, of a patient, in particular a therapeutic pneumoperitoneum.

In a yet another particular embodiment 27, the present invention pertains to a syringe, especially a single-use syringe, according to embodiment 25 as a container (9) in a sterile and closed prefilled drug containing (medical) device system, or in a kit comprising said sterile and closed prefilled drug containing (medical) device system, for administering, preferably for use in a treatment for administering, at least one pharmaceutically active substance (drug) by pressurized aerosol therapy (PAT) in a hollow organ, or preferably in a body cavity, of a patient, in particular a therapeutic pneumoperitoneum, or a syringe (or use thereof), especially a single-use syringe, according to embodiment 26 as a container (9) for use in the preparation of such a sterile and closed prefilled drug containing (medical) device system, or in a kit comprising said sterile and closed prefilled drug containing (medical) device system,
wherein the said final drug containing (medical) device system, or said final drug containing (medical) device system of the kit, is comprising a flexible package (100, 101), comprising:
- at least one syringe, especially a single-use syringe, as container (9) comprising at least one pharmaceutically active substance (70) in powder, lyophilisate, solution, liquid, gel or other form (suspension, emulsion), preferably in solution, liquid, gel form, and being provided with breakable, an openable, or perforable cap (60); and
- a bag (2) of liquid diluent (50),
   ∘ wherein the liquid diluent (50) is preferably a physiologically acceptable aqueous diluent;
   ∘ wherein the bag (2) is optionally equipped with at least one drain tube (4) provided with a closing device (5);
   ∘ and wherein the bag (2) is equipped with a mixing tube (6), optionally equipped with an openable closure (7), and which optionally ends with a perforation device (8), or which optionally ends with a coupling and perforation device (8), for a syringe, especially a single-use syringe, as a container (9), comprising the pharmaceutically active substance (70) in powder, lyophilisate, solution, liquid, gel or other material (suspension, emulsion), preferably in solution, liquid, gel form, and which syringe, especially single-use syringe, as container (9) is provided with breakable, an openable, or perforable cap (60);
   and
   (i) wherein in the said final drug containing (medical) device system, the said package (100, 101) optionally is comprising at least one flexible airtight sterile casing (12), containing said syringe, especially a single-use syringe, as container (9) of the said pharmaceutically active substance, and said perforation device (8), optionally the said coupling and perforation device (8), said container (9) being housed in the casing (12), optionally in a coupling position with the said optional coupling and perforation device (8); and wherein the said syringe, especially single-use syringe, as the container (9) is manually maneuverable from the outside of the optional casing (12) in order to be opened inside the closed optional casing (12), optionally is manually maneuverable from the outside of the optional casing (12) up to a perforating position of said cap (60) through the same perforation device (8), optionally through the same coupling and perforation device (8);
      or
   (ii) wherein in case of a syringe, especially single-use syringe, as the container (9), the said package (100, 101) is devoid of a flexible airtight sterile casing (12) the syringe, especially single-use syringe, as the container (9) is already fixed to the bag (2) in a coupling position with the said optional coupling and perforation device (8);
      and
   whereupon after opening of the said syringe, especially single-use syringe, as the container (9) and of the said closure (7), and selectively mixing (i.e. dissolving, reconstituting) the at least one pharmaceutically active substance (drug) (70) with the liquid diluent (50) through the mixing tube (6), a solution of the at least one pharmaceutically active substance (drug) (70) in the liquid diluent (50) is provided for administering by pressurized aerosol therapy (PAT) in a hollow organ, or preferably in a body cavity of a patient.

Any features as defined in any of the embodiments 2 to 19 are equally applicable for the embodiments 25 to 27 pertaining to embodiments of the invention involving a syringe, especially a single-use syringe. Also, the embodiments 25 to 27 pertaining to embodiments of the invention involving a syringe, especially a single-use syringe, may be applied in a kit as defined in the embodiments 20 to 21, and as well may be applied in a method of treatment (not claimed) as defined in the embodiments 23 to 24. In a further embodiment a pump, in particular a micro-pump, for transporting the pressurized substance into the nozzle system is connectable to the nozzle system, such that the substance is convertible into the aerosol by means of a needle nozzle or a needle valve. According to this embodiment of the invention comprising a micro-pump for transporting the liquid drug substance, the drug substance may be a suitable liquid, which may include additional substances for various purposes such as cancer treatment, treatment of infection, and the prevention of adhesions. The micro-pump can be formed in one embodiment by a syringe, having a piston and a cylinder, wherein the syringe can be loaded in a device, and wherein said device compresses the piston, thereby generating a fluid pressure.

According to one embodiment an insufflator is connectable to the trocar system, said insufflator providing the insufflation gas for forming a hollow space, in particular a therapeutic pneumoperitoneum, in a patient at 12 to 15 mm Hg.

According to one embodiment of the invention having a nozzle system, to which the micro-pump is connected. The micro-pump and the nozzle system are connected to each other in a fluid conductive manner, so that a drug substance, which is transported in a pressurized manner by the micro-pump, is atomized to a mist-like aerosol by needle nozzle. In addition, the aerosol may be electrostatically charged by a voltage applied to the trocar system. The substance may include in particular drugs suitable for chemotherapy, such as cytostatics, such as doxorubicin, Cisplatin or other chemotherapeutic agents, or others as described herein above.

The invention is particularly useful in combination and/or can be performed in connection with a method and device for aerosol therapy as described in the DE 10 2013 109 896 A1.

The embodiments of the invention can be used in a method (not claimed) for the directed application of a substance in a hollow space, such as a hollow organ, of a body cavity, in particular, a therapeutic pneumoperitoneum, especially by a pressurized aerosol (chemo)therapy as any one of those defined herein above, comprising at least one step of:
(i) inserting a trocar sleeve a trocar system with a trocar sleeve;
(ii) supplying the trocar sleeve with an insufflation gas;
(iii) penetration of the trocar sleeve with a nozzle system, which in its interior forms a lumen, with a proximal end and a distal end, wherein the nozzle system has a needle nozzle fixed at the distal end with the lumen and is guided by the trocar sleeve;
(iv) generating an aerosol in the hollow space, such as a hollow organ, of a body cavity, via the needle nozzle.

According to one embodiment of the method, mainly carbon dioxide is provided as insufflation gas.

The invention further pertains to and can be illustrated by the following exemplary and representative for use in method of treatment embodiments, wherein the methods are not separately claimed.

For use in a method embodiment A: A method of treating a hollow organ, or preferably in a body cavity, of a patient, in particular a therapeutic pneumoperitoneum, wherein the method comprises
providing a sterile and closed prefilled drug containing (medical) device system for administering, preferably for use in administering, at least one pharmaceutically active substance (drug) by pressurized aerosol therapy (PAT) in a hollow organ, or in a body cavity, of a patient, comprising a flexible package (100, 101) as defined above, and in any of the claims 1 to 9; optionally as part of a kit as defined above, and in any of the claims 10 to 12;
   and
mixing the at least one pharmaceutically active substance (70) comprised in powder, lyophilisate, solution, liquid, gel or other form (suspension, emulsion) in the at least one container (9) being provided with breakable, an openable, or perforable cap (60), with the liquid diluent (50) comprised in the bag (2), to result in a solution of the at least one pharmaceutically active substance (70) in the liquid diluent (50);
and then generating a pressurized aerosol from the said solution of the at least one pharmaceutically active substance (70) in the liquid diluent (50), and thereby administering the said solution of the at least one pharmaceutically active substance (drug) by pressurized aerosol therapy (PAT) in a hollow organ, or preferably in a body cavity, of a patient, in particular a therapeutic pneumoperitoneum.

For use in a method embodiment B: A method of treating a hollow organ, or preferably in a body cavity, of a patient, in particular a therapeutic pneumoperitoneum, wherein the method comprises
providing a sterile and closed prefilled drug containing (medical) device system for administering, preferably for use in administering, at least one pharmaceutically active substance (drug) by pressurized aerosol therapy (PAT) in a hollow organ, or in a body cavity, of a patient, comprising a flexible package (100, 101) as defined above, and in any of the claims 1 to 9; optionally as part of a kit as defined above, and in any of the claims 10 to 12;
   and
mixing the at least one pharmaceutically active substance (70) comprised in powder, lyophilisate, solution, liquid, gel or other form (suspension, emulsion) in the at least one container (9) being provided with breakable, an openable, or perforable cap (60), with the liquid diluent (50) comprised in the bag (2), to result in a solution of the at least one pharmaceutically active substance (70) in the liquid diluent (50);
and then generating a pressurized aerosol from the said solution of the at least one pharmaceutically active substance (70) in the liquid diluent (50), and thereby administering the said solution of the at least one pharmaceutically active substance (drug) by pressurized aerosol therapy (PAT) in a hollow organ, or preferably in a body cavity, of a patient, in particular a therapeutic pneumoperitoneum;
wherein the said generated pressurized aerosol is administered by means of an application lance and/or injection lance with nozzle, preferably a single use (or disposable) application lance and/or injection lance with nozzle, for applying, i.e. for use in administering, the at least one pharmaceutically active substance (drug) by pressurized aerosol therapy (PAT) in a hollow organ, or in a body cavity, of a patient.

For use in a method embodiment C: The method of treating a hollow organ, or preferably in a body cavity, of a patient, in particular a therapeutic pneumoperitoneum, according to embodiment A for use or embodiment B for use,
wherein as a hollow space the body cavity is selected from the group consisting of each hollow space in the body that is lined by mesothelium and epithelium, self-contained or connected with other cavities or the outside world, such as the abdomen, peritoneal cavity, thoracic cavity, luminal cavity, vesical cavity, cranial cavity, nasal cavity, oral cavity, pharynx, subarachnoid spaces, or joint cavities; preferably wherein the body cavity is selected from the group consisting of the peritoneal cavity (peritoneum), thoracic cavity (thorax), luminal cavity (lumen), vesical cavity (vesicle), uterus;
or wherein as hollow space the hollow organ is selected from the group consisting of such as the cavity of the heart, blood vessels, bile duct, urinary tract, gastrointestinal tract, uterus or brain ventricles.

For use in a method embodiment D: The method of treating a hollow organ, or preferably in a body cavity, of a patient, in particular a therapeutic pneumoperitoneum, according to embodiment A for use or embodiment B for use, wherein the pressurized aerosol therapy (PAT) is selected from the group consisting of pressurized intra-peritoneal aerosol chemotherapy (PIPAC), pressurized intra-thoracic aerosol chemotherapy (PITAC), pressurized intra-luminal aerosol chemotherapy (PILAC), and pressurized intra-vesical aerosol chemotherapy (PIVAC), pressurized intra-rectal aerosol chemotherapy (PIRAC). For use in a method embodiment E: The method of treating a hollow organ, or preferably in a body cavity, of a patient, in particular a therapeutic pneumoperitoneum, according to embodiment A for use or embodiment B for use, wherein the method is in a treatment of cancer and/or tumor treatable by rinsing and/or perfusion, preferably in a treatment of cancer and/or tumor, including but not limited to as well as metastatic forms thereof, selected from the group consisting of cancer/tumor of the peritoneum, cancer/tumor of the urinary tract, in particular bladder cancer, ovarian cancer, cancer/tumor of the colorectum, and most preferably of cancer/tumor of the peritoneum; and/or in a treatment of cancer and/or tumor selected from the group consisting of in particular colorectal cancer, cervical cancer, uterine cancer, bladder cancer, lung cancer, esophageal cancer, gastric cancer, pancreatic cancer, peritoneal cancer, and prostate cancer, as well as metastatic forms thereof.

### In the drawings:

Further advantageous embodiments of the invention and exemplary embodiments thereto, as compared to the prior art (see e.g., Fig. 2) will be explained in more detail below in conjunction with the accompanying drawings. Similarly functioning parts or components are partially provided with the same reference numerals. For better understanding in the following figures the respective features are schematic and exaggerated and are not shown to scale.
FIG. 1 shows, as an exemplification of the context of the invention, a schematic view of a typical PIPAC setting.
FIG. 2 shows a schematic view of a typical cartridge as it is currently used in the prior art, for example, in a typical PIPAC setting, for individually preparing, e.g. reconstituting, a dosage to be administered by a pressurized aerosol therapies (PAT), by a (hospital) pharmacy under (more or less) sterile and environmental conditions.
FIG. 3 to 7 show, as an exemplification but not limiting, simplified drug and device systems according to the present invention, for the directed application of a substance by means of a pressurized aerosol therapy (PAT) into a hollow space, such as a hollow organ, or into a body cavity of a patient, i.e. a mammalian patient.

The patient may, as mentioned, be a human. The invention, however, is not limited to humans but can be applied to other organisms. The term patient shall therefore describe organism being that requires medical treatment and can be treated with the embodiments according to the invention.

An example of a hollow space target in the context of the present invention is the abdominal cavity (abdomen): Peri- and postoperative pain treatment, destruction of tumor cells (gastric cancer, ovarian cancer, or other cancers), destruction of bacteria (peritonitis), prevention and treatment of peritoneal carcinomatosis, prevention of adhesions. Chest (thorax): Peri- and postoperative pain treatment, destruction of tumor cells (lung cancer, pleural cancer, other cancers), destruction of bacteria (pleuritis), prevention and treatment of pleural carcinosis. Further applications can be found in endoscopic surgeries in a non-preformed space (e.g. in the retroperitoneum).

Variations and modifications from the described embodiments exist. Any number disclosed herein should be construed to mean approximate, regardless of whether the word "about" or "approximately" is used in describing the number. The appended claims intend to cover all those modifications and variations as falling within the scope of the invention.

## Claims

1. Kit for use in a treatment of administering at least one pharmaceutically active substance by PAT (pressurized aerosol therapy) in a hollow organ or in a body cavity, of a patient, comprising:
(a) a sterile and closed prefilled drug containing device system for use in administering at least one pharmaceutically active substance by PAT in a hollow organ or in a body cavity of a patient, comprising a flexible package (100, 101), comprising:
- at least one container (9) comprising at least one pharmaceutically active substance (70) in powder, lyophilisate, solution, liquid, gel or suspension or emulsion form, and being provided with breakable, an openable, or perforable cap (60); and
- a bag (2) of liquid diluent (50), connectable to an aerosol generating means, for use with an application lance and/or injection lance with nozzle,
∘ wherein the liquid diluent (50) is a physiologically acceptable aqueous diluent;
∘ and wherein the bag (2) is equipped with a mixing tube (6);
and
wherein said package (100, 101) comprises at least one flexible airtight sterile casing (12), containing said container (9), and said perforation device (8); and
wherein the said container (9) is manually maneuverable from the outside of the casing (12) in order to be opened inside the closed casing (12); and
whereupon after opening of the said container (9) and of the said closure (7), and selectively mixing by dissolving or reconstituting the at least one pharmaceutically active substance (70) with the liquid diluent (50) through the mixing tube (6), a solution of the at least one pharmaceutically active substance (70) in the liquid diluent (50) is provided for administering by PAT in a hollow organ or in a body cavity of a patient;
and
(b) an application lance and/or injection lance with nozzle;
and/or
(c) a pumping system, preferably a micro-pump, for use with an application lance and/or injection lance with nozzle for use in administering the at least one pharmaceutically active substance by PAT in a hollow organ or in a body cavity of a patient.

2. The kit according to claim 1 further comprising:
(d) tubes and/or connectors, for use with the (a) the sterile and closed prefilled drug containing device system, (b) the application lance and/or injection lance with nozzle, and/or (c) the pumping system, preferably a micro-pump.

3. The kit according to any one of claims 1 and 2, wherein:
- the bag (2) is optionally equipped with at least one drain tube (4) provided with a closing device (5); preferably wherein the optional at least one drain tube (4), after removal of the closing device (5), serves as connector to an aerosol generating means, for use with an application lance and/or injection lance with nozzle; and/or
- the bag (2) is equipped with an openable closure (7), which optionally ends with a perforation device (8), or which optionally ends with a coupling and perforation device (8), for the container (9), wherein optionally the container (9) being housed in the casing (12) in a coupling position with the coupling and perforation device (8); and/or
- the bag (2) is equipped with an openable closure (7), which ends with a perforation device (8), or which optionally ends with a coupling and perforation device (8), the container (9) is provided with breakable, an openable, or perforable cap (60) and the container (9) is manually maneuverable from the outside of the casing (12) up to a perforating position of said cap (60) through the perforation device (8), optionally through the coupling and perforation device (8).

4. The kit according to any one of claims 1 to 3, comprising the application lance and/or injection lance with nozzle, preferably a single use application lance and/or injection lance with nozzle.

5. The kit according to any one of claims 1 to 4, wherein the kit is a single use kit.

6. The kit according any one of claims 1 to 3, wherein under (c) the pumping system is a micro-pump.

7. The kit according to any one of claims 1 to 6, further comprising at least one multiple-way valve (10) connectable to or connecting the interior space of bag (2) and the interior space of at least one flexible airtight sterile casing (12) of said package (100, 101), preferably wherein the multiple-way valve (10) is selected from the group of a three-way valve, a three-way slipper valve, a three-way cock, a three-way stopcock.

8. The kit according to any one of claims 1 to 7, comprising in the at least a flexible airtight sterile casing (12), more than one of container (9), preferably two to three containers (9a, 9b, 9c), more preferably two containers (9a, 9b), each container comprising independently at least one pharmaceutically active substance.

9. The kit according to any one of claims 1 to 8, wherein the container (9) is a bottle (9), the kit comprising the coupling and perforation device (8), and wherein the bag is equipped with a openable closure (7), which ends with the coupling and perforation device (8), the container ()) being provided with a breakable, openable, or perforable cap (6), wherein the coupling and perforation device (8) comprises a first element slidably coupled with a second element (22) and movable between said coupling position of the bottle (9) and said perforating position of the cap (60) of the bottle (9), in which said first element comprises a ring (23) from which at least two flaps (24) equipped with notches (25, 26) suitable to accommodate the bottle (9) in the coupling position, vertically branch off, and in that said openable closure (7) is surmounted by a circular base (28) from which pairs of further flaps (27), in turn equipped with notches (52) suitable to accommodate the bottle (9) in the perforating position of the cap (60), perimetrically and vertically side by side branch off, said second element (22) having gaps (30) between said couples of further flaps (27) in correspondence of said at least two flaps (24), said gaps (30) being suitable to accommodate the at least two flaps (24) with the bottle (9) in the perforating position of the cap (60).

10. The kit according to any of claims 1 to 9, wherein the at least one pharmaceutically active substance (70) is for use in a treatment of cancer and/or tumor treatable by rinsing and/or perfusion, preferably in a treatment of cancer and/or tumor selected from the group consisting of cancer/tumor of the peritoneum, cancer/tumor of the urinary tract, in particular bladder cancer, ovarian cancer, cancer/tumor of the colorectum, and most preferably of cancer/tumor of the peritoneum; and/or in a treatment of cancer and/or tumor selected from the group consisting of in particular colorectal cancer, cervical cancer, uterine cancer, bladder cancer, lung cancer, esophageal cancer, gastric cancer, pancreatic cancer, peritoneal cancer, and prostate cancer, as well as metastatic forms thereof.

11. The kit according to any one of claims 1 to 9, wherein the at least one pharmaceutically active substance (70) is for use in administering by pressurized aerosol therapy (PAT) in a hollow organ, or in a body cavity, of a patient for non-systemic (therapeutic) efficacy, preferably for local and/or topical (therapeutic) efficacy in a hollow organ, or in a body cavity, of a patient.

12. The kit according to any one of claims 1 to 9, wherein the at least one pharmaceutically active substance (70) is selected from
(a) the group consisting of antitumor, anticancer, cytostatic, antinecrotic, antimicrobial, antimycotic, antibacterial, antiviral, antiseptic, antibiotic substances or a combination thereof; and/or
(b) the group consisting of anticancer, antitumor, cytostatic substances or a combination thereof.

13. The kit according to claim 12, wherein the at least one pharmaceutically active substance (70) is selected from the group consisting of anticancer, antitumor, cytostatic substances or a combination thereof is selected from the group consisting of aflibercept, amsacrin, alemtuzumab, arsene trioxide, asparaginase, azacitidine, bendamustin hydrochloride, bevacizumab, bleomycin, blinatumomab, bortezomib, busulfan, cabacitaxel, carboplatin, carfilzomib, carmustine, 2 CdA /cladribin, cetuximab, cidofovir, cisplatin, clofarabin, cyclophosphamide, cytarabin, dacarbacin, dactinomycin, daratumab, daunorubicin, decitabin, docetaxel, doxorubicin, liposomales doxorubicin, eribulin mesilate, epirubicin, etoposid, fludarabin, 5-flourouracil, foscarnet, gemicitabin, glancyclovir, idarubicin, ifosfamide, ipilmumab, irinotecan, melphalan, methotrexat, mifamurtid, mitomycin, mitoxantron, nelarabin, nivolumab, obinituzumab ofatumumab, oxaliplatin, paclitaxel, panitumab, pemetrexed, pertuzumab, prembolizumab, ramucirumab, rituximab, romidepsin, thiotepa, topotecan, trabectedin, trastuzumab, trastuzumab-emtansin, treosulfan, vinblastine, vincristin, vindesin, vinflunin, vinorelbin.

14. The kit according to claim 12, wherein the at least one pharmaceutically active substance (70) is selected from one of:
(c) a combination of doxorubicin, cisplatin, oxaliplatin, topotecan, and paclitaxel;
(d) a combination of pemetrexed, bendamustin, topotecan, and carboplatin;
(e) a combination of irinotecan and docetaxel;
(f) a combination of doxorubicin, mitomycin, and epirubicin;
(g) a combination of docetaxel, oxaliplatin, paclitaxel, and vinorelbin.

## Patentansprüche

1. Kit zur Verwendung bei einer Behandlung zur Verabreichung von mindestens einer pharmazeutisch aktiven Substanz mittels PAT (Druckaerosoltherapie) in ein Hohlorgan oder eine Körperhöhle eines Patienten, umfassend:
(a) ein steriles und geschlossenes, vorgefülltes Arzneimittel enthaltendes Vorrichtungssystem zur Verwendung bei der Verabreichung von mindestens einer pharmazeutisch aktiven Substanz mittels PAT in ein Hohlorgan oder in eine Körperhöhle eines Patienten, umfassend eine flexible Verpackung (100, 101), die Folgendes umfasst:
- mindestens einen Behälter (9), der mindestens eine pharmazeutisch wirksame Substanz (70) in Pulver-, Lyophilisat-, Lösungs-, Flüssigkeits-, Gel- oder Suspensions- oder Emulsionsform enthält, und mit einer abbrechbaren, zu öffnenden oder durchstechbaren Kappe (60) versehen ist; und
- eine Tasche (2) mit flüssigem Verdünnungsmittel (50), der an eine Aerosolerzeugungseinrichtung anschließbar ist, zur Verwendung mit einer Applikationslanze und/oder Injektionslanze mit Düse,
∘ wobei das flüssige Verdünnungsmittel (50) ein physiologisch verträgliches wässriges Verdünnungsmittel ist;
∘ und wobei die Tasche (2) mit einem Mischröhrchen (6) ausgestattet ist; und
wobei die Verpackung (100, 101) mindestens ein flexibles, luftdichtes, steriles Gehäuse (12) umfasst, die den Behälter (9) und die Perforationsvorrichtung (8) enthält; und
wobei der Behälter (9) von der Außenseite des Gehäuses (12) aus manuell manövrierbar ist, um innerhalb des geschlossenen Gehäuses (12) geöffnet zu werden; und
woraufhin nach dem Öffnen des Behälters (9) und des Verschlusses (7) und dem selektiven Mischen durch Auflösen oder Rekonstituieren der mindestens einen pharmazeutisch aktiven Substanz (70) mit dem flüssigen Verdünnungsmittel (50) durch das Mischröhrchen (6) eine Lösung der mindestens einen pharmazeutisch aktiven Substanz (70) in dem flüssigen Verdünnungsmittel (50) zur Verabreichung mittels PAT in ein Hohlorgan oder in eine Körperhöhle eines Patienten bereitgestellt wird;
und
(b) eine Applikationslanze und/oder Injektionslanze mit Düse;
und/oder
(c) ein Pumpsystem, vorzugsweise eine Mikropumpe, zur Verwendung mit einer Applikationslanze und/oder Injektionslanze mit Düse zur Verwendung bei der Verabreichung der mindestens einen pharmazeutisch aktiven Substanz mittels PAT in ein Hohlorgan oder in eine Körperhöhle eines Patienten.

2. Das Kit gemäß Anspruch 1 weiterhin umfassend:
(d) Schläuche und/oder Verbindungsstücke zur Verwendung mit dem (a) dem sterilen und geschlossenen, vorgefüllten Arzneimittel enthaltenden Vorrichtungssystem, (b) der Applikationslanze und/oder Injektionslanze mit Düse und/oder (c) dem Pumpsystem, vorzugsweise einer Mikropumpe.

3. Das Kit gemäß einem der Ansprüche 1 und 2, wobei:
- die Tasche (2) optional mit mindestens einem Ablassröhrchen (4) ausgestattet ist, der mit einer Verschlussvorrichtung (5) versehen ist; wobei vorzugsweise das optionale mindestens eine Ablassröhrchen (4) nach Entfernen der Verschlussvorrichtung (5) als Anschluss für eine Aerosolerzeugungseinrichtung zur Verwendung mit einer Applikationslanze und/oder Injektionslanze mit Düse dient; und/oder
- die Tasche (2) mit einem öffenbaren Verschluss (7) ausgestattet ist, der wahlweise mit einer Perforationsvorrichtung (8) endet oder der wahlweise mit einer Verbindungs- und Perforationsvorrichtung (8) für den Behälter (9) endet, wobei wahlweise der Behälter (9) in einer Verbindungsposition mit der Verbindungs- und Perforationsvorrichtung (8) in in dem Gehäuse (12) untergebracht ist; und/oder
- die Tasche (2) mit einem zu öffnenden Verschluss (7) ausgestattet ist, der mit einer Perforationsvorrichtung (8) endet oder der wahlweise mit einer Verbindungs- und Perforationsvorrichtung (8) endet, der Behälter (9) mit einer zerbrechlichen, zu öffnenden oder perforierbaren Kappe (60) versehen ist und der Behälter (9) manuell von der Außenseite des Gehäuses (12) durch die Perforationsvorrichtung (8), wahlweise durch die Verbindungs- und Perforationsvorrichtung (8), bis zu einer Perforationsposition der genannten Kappe (60) manövrierbar ist.

4. Das Kit gemäß einem der Ansprüche 1 bis 3, umfassend die Applikationslanze und/oder Injektionslanze mit Düse, vorzugsweise eine Applikationslanze und/oder Injektionslanze mit Düse zum einmaligen Gebrauch.

5. Das Kit gemäß einem der Ansprüche 1 bis 4, wobei es sich bei dem Kit um ein Kit zur einmaligen Verwendung handelt.

6. Das Kit gemäß einem der Ansprüche 1 bis 3, wobei gemäß (c) das Pumpsystem eine Mikropumpe ist.

7. Das Kit gemäß einem der Ansprüche 1 bis 6, das weiterhin mindestens ein Mehrwegeventil (10) umfasst, das mit dem Innenraum der Tasche (2) und dem Innenraum des mindestens einem flexiblen, luftdichten, sterilen Gehäuse (12) der Verpackung (100, 101) verbindbar ist oder diese verbindet, wobei das Mehrwegeventil (10) vorzugsweise aus der Gruppe bestehend aus einem Dreiwegeventil, einem Dreiwege-Schiebeventil, einem Dreiwegehahn oder einem Dreiwege-Absperrhahn ausgewählt ist.

8. Das Kit gemäß einem der Ansprüche 1 bis 7, das in dem mindestens einen flexiblen, luftdichten, sterilen Gehäuse (12) mehr als einen Behälter (9), vorzugsweise zwei bis drei Behälter (9a, 9b, 9c), bevorzugter zwei Behälter (9a, 9b) enthält, wobei jeder Behälter unabhängig mindestens eine pharmazeutisch wirksame Substanz enthält.

9. Das Kit nach einem der Ansprüche 1 bis 8, wobei der Behälter (9) eine Flasche (9) ist, das Kit die Verbindungs- und Perforationsvorrichtung (8) umfasst und wobei die Tasche mit einem zu öffnenden Verschluss (7) ausgestattet ist, der mit der Verbindungs- und Perforationsvorrichtung (8) endet, wobei der Behälter (9) mit einer zerbrechlichen, zu öffnenden oder durchstechbaren Kappe (60) versehen ist, wobei die Verbindungs- und Perforationsvorrichtung (8) ein erstes Element umfasst, das verschiebbar mit einem zweiten Element (22) gekoppelt ist und zwischen der Verbindungsposition der Flasche (9) und der Perforationsposition der Kappe (60) der Flasche (9) beweglich ist, wobei das erste Element einen Ring (23) umfasst, von dem mindestens zwei Klappen (24) vertikal abzweigen, die mit Aussparrungen (25, 26) ausgestattet sind, die geeignet sind, die Flasche (9) in der Verbindungsposition aufzunehmen, und dass der zu öffnende Verschluss (7) von einer kreisförmigen Basis (28) überragt wird, von der Paare weiterer Klappen (27) abzweigen, die wiederum mit Aussparrungen (52) ausgestattet sind, die geeignet sind, die Flasche (9) in der Perforationsposition der Kappe (60) aufzunehmen, und die perimetrisch und vertikal Seite an Seite abzweigen, wobei das zweite Element (22) Aussparrungen (30) zwischen den Paaren weiterer Klappen (27) in Übereinstimmung mit den mindestens zwei Klappen (24) aufweist, wobei die Lücken (30) geeignet sind, die mindestens zwei Klappen (24) mit der Flasche (9) in der Perforationsposition der Kappe (60) aufzunehmen.

10. Kit nach einem der Ansprüche 1 bis 9, wobei die mindestens eine pharmazeutisch wirksame Substanz (70) zur Verwendung bei einer Behandlung von Krebs und/oder Tumoren bestimmt ist, die durch Spülung und/oder Perfusion behandelbar sind, vorzugsweise bei einer Behandlung von Krebs und/oder Tumoren ausgewählt aus der Gruppe bestehend aus Krebs/Tumor des Bauchfells, Krebs/Tumor der Harnwege, insbesondere Blasenkrebs, Eierstockkrebs, Krebs/Tumor des Dickdarms, und am bevorzugtesten von Krebs/Tumor des Bauchfells; und/oder bei einer Behandlung von Krebs und/oder Tumoren ausgewählt aus der Gruppe bestehend aus insbesondere Dickdarmkrebs, Gebärmutterhalskrebs, Gebärmutterkrebs, Blasenkrebs, Lungenkrebs, Speiseröhrenkrebs, Magenkrebs, Bauchspeicheldrüsenkrebs, Bauchfellkrebs und Prostatakrebs sowie metastasierten Formen davon.

11. Das Kit gemäß einem der Ansprüche 1 bis 9, wobei die mindestens eine pharmazeutisch aktive Substanz (70) zur Verwendung bei der Verabreichung mittels Druckaerosoltherapie (PAT) in ein Hohlorgan oder in eine Körperhöhle eines Patienten zur nichtsystemischen (therapeutischen) Wirksamkeit, vorzugsweise zur lokalen und/oder topischen (therapeutischen) Wirksamkeit in einem Hohlorgan oder in einer Körperhöhle eines Patienten bestimmt ist.

12. Das Kit gemäß einem der Ansprüche 1 bis 9, wobei die mindestens eine pharmazeutisch aktive Substanz (70) ausgewählt ist aus
(a) der Gruppe bestehend aus Antitumor-, Antikrebs-, Zytostatika-, Antinekrotika-, antimikrobiellen, antimykotischen, antibakteriellen, antiviralen, antiseptischen, antibiotischen Substanzen oder einer Kombination davon; und/oder
(b) der Gruppe bestehend aus Antikrebs-, Antitumor-, Zytostatika-Substanzen oder einer Kombination davon.

13. Das Kit gemäß Anspruch 12, wobei die mindestens eine pharmazeutisch aktive Substanz (70) ausgewählt ist aus der Gruppe bestehend aus Antikrebs-, Antitumor-, Zytostatika-Substanzen oder eine Kombination davon ausgewählt ist aus der Gruppe bestehend aus Aflibercept, Amsacrin, Alemtuzumab, Arsentrioxid, Asparaginase, Azacitidin, Bendamustinhydrochlorid, Bevacizumab, Bleomycin, Blinatumomab, Bortezomib, Busulfan, Cabacitaxel, Carboplatin, Carfilzomib, Carmustin, 2 CdA /Cladribin, Cetuximab, Cidofovir, Cisplatin, Clofarabin, Cyclophosphamid, Cytarabin, Dacarbacin, Dactinomycin, Daratumab, Daunorubicin, Decitabin, Docetaxel, Doxorubicin, liposomales Doxorubicin, Eribulinmesilat, Epirubicin, Etoposid, Fludarabin, 5-Fluorouracil, Foscarnet, Gemicitabin, Glancyclovir, Idarubicin, Ifosfamid, Ipilmumab, Irinotecan, Melphalan, Methotrexat, Mifamurtid, Mitomycin, Mitoxantron, Nelarabin, Nivolumab, Obinituzumab, Ofatumumab, Oxaliplatin, Paclitaxel, Panitumab, Pemetrexed, Pertuzumab, Prembolizumab, Ramucirumab, Rituximab, Romidepsin, Thiotepa, Topotecan, Trabectedin, Trastuzumab, Trastuzumab-Emtansin, Treosulfan, Vinblastin, Vincristin, Vindesin, Vinflunin, Vinorelbin.

14. Das Kit gemäß Anspruch 12, wobei die mindestens eine pharmazeutisch aktive Substanz (70) ausgewählt ist aus einem der Folgenden:
(c) eine Kombination aus Doxorubicin, Cisplatin, Oxaliplatin, Topotecan und Paclitaxel;
(d) eine Kombination aus Pemetrexed, Bendamustin, Topotecan und Carboplatin;
(e) eine Kombination aus Irinotecan und Docetaxel;
(f) eine Kombination aus Doxorubicin, Mitomycin und Epirubicin;
(g) eine Kombination aus Docetaxel, Oxaliplatin, Paclitaxel und Vinorelbin.

## Revendications

1. Kit destiné à être utilisé dans un traitement pour l'administration au moins une substance pharmaceutiquement active au moyen de PAT (thérapie par aérosol sous pression) dans un organe creux ou une cavité corporelle d'un patient, comprenant :
(a) un système de dispositif stérile et fermé contenant un médicament prérempli destiné à être utilisé pour administrer au moins une substance pharmaceutiquement active au moyen de PAT dans un organe creux ou une cavité corporelle d'un patient, comprenant un emballage flexible (100, 101) qui comprend :
- au moins un récipient (9) qui contient au moins une substance pharmaceutiquement active (70) sous forme de poudre, de lyophilisat, de solution, de liquide, de gel, de suspension ou d'émulsion et étant pourvu d'un capuchon cassable, ouvrable ou perforable (60); et
- une poche (2) de diluant liquide (50), connectable à un dispositif générateur d'aérosol, à utiliser avec une lance d'application et/ou une lance d'injection avec buse,
∘ dans lequel le diluant liquide (50) est un diluant aqueux physiologiquement acceptable;
∘ et dans lequel la poche (2) est équipé d'un tube mélangeur (6);
et
dans lequel l'emballage (100, 101) comprend au moins un boîtier flexible, étanche à l'air et stérile (12) qui contient le récipient (9) et le dispositif de perforation (8); et
dans lequel le récipient (9) pouvant être manoeuvré manuellement depuis l'extérieur du boîtier (12) pour être ouvert à l'intérieur du boîtier fermé (12); et
après quoi, après ouverture du récipient (9) et de la fermeture (7) et la mixtion sélective par dissolution ou reconstitution de l'au moins une substance pharmaceutiquement active (70) avec le diluant liquide (50) à travers le tube de mélange (6), une solution de l'au moins une substance pharmaceutiquement active (70) est préparer dans le diluant liquide (50) pour administration au moyen de PAT dans un organe creux ou dans une cavité corporelle d'un patient;
et
(b) une lance d'application et/ou une lance d'injection avec buse;
et/ou
(c) un système de pompe, de préférence une micropompe, destiné à être utilisé avec une lance d'application et/ou une lance d'injection avec buse destinée à être utilisée pour administrationde l'au moins une substance pharmaceutiquement active au moyen de PAT dans un organe creux ou dans une cavité corporelle d'un patient.

2. Le kit selon la revendication 1, comprenant en outre:
(d) tubes et/ou connecteurs destinés à être utilisés avec (a) le système de dispositif stérile et fermé contenant un médicament prérempli, (b) la lance d'application et/ou la lance d'injection avec buse et/ou (c) le système de pompe, de préférence une micropompe.

3. Le kit selon l'une quelconque des revendications 1 et 2, dans lequel:
- la poche (2) est facultativement équipé d'au moins un tuyau de vidange (4) qui est pourvu d'un dispositif de fermeture (5); dans lequel, de préférence, le au moins tuyau de vidange facultatif (4) après avoir retiré le dispositif de fermeture (5) servent de connexion pour un dispositif générateur d'aérosol destiné à être utilisé avec une lance d'application et/ou une lance d'injection avec buse après que le dispositif de fermeture (5) ait été retiré; et/ou
- la poche (2) est équipé d'une fermeture ouvrable (7), qui se termine facultativement par un dispositif de perforation (8) ou qui se termine facultativement par un dispositif de couplage et de perforation (8) pour le récipient (9), grâce auquel le récipient (9) est logé dans une position de couplage avec le dispositif de couplage et de perforation (8) dans le boîtier (12); et/ou
- la poche (2) est équipé d'une fermeture ouvrable (7) qui se termine par un dispositif de perforation (8) ou qui se termine facultativement par un dispositif de couplage et de perforation (8), le récipient (9) est pourvu d'un capuchon cassable, ouvrable ou perforable (60) et le récipient (9) peut être manoeuvré manuellement depuis l'extérieur du boîtier (12) à travers le dispositif de perforation (8), facultativement à travers le dispositif de couplage et de perforation (8), jusqu'à une position de perforation dudit capuchon (60).

4. Le kit selon l'une quelconque des revendications 1 à 3, comprenant la lance d'application et/ou la lance d'injection avec buse, de préférence une lance d'application et/ou la lance d'injection avec buse à usage unique.

5. Le kit selon l'une quelconque des revendications 1 à 4, dans lequel le kit est un kit à usage unique.

6. Le kit selon l'une quelconque des revendications 1 à 3, dans lequel en (c) le système de pompage est une micropompe.

7. Le kit selon l'une quelconque des revendications 1 à 6, comprenant en outre au moins une valve multivoies (10) qui peuvent être connectées à l'intérieur de la poche (2) et à l'intérieur de l'au moins un boîtier flexible, étanche à l'air et stérile (12) de l'emballage (100, 101) peuvent être connectés ou qui les connecte, dans lequel la valve multivoies (10) étant de préférence choisie dans le groupe constitué d'une valve à trois voies, d'un tiroir à trois voies, d'un robinet à trois voies ou un robinet d'arrêt à trois voies.

8. Le kit selon l'une quelconque des revendications 1 à 7, qui contient dans le au moins un boîtier flexible, étanche à l'air et stérile (12), plus d'un récipient (9), de préférence deux à trois récipients (9a, 9b, 9c), plus de préférence deux récipients (9a, 9b), chaque récipient contenant indépendamment au moins une substance pharmaceutiquement active.

9. Le kit selon l'une quelconque des revendications 1 à 8, dans lequel le récipient (9) est une bouteille (9), le kit comprend le dispositif de couplage et de perforation (8), et dans lequel la poche est équipé d'une fermeture ouvrable (7) qui se termine par le dispositif de couplage et de perforation (8), dans lequel le récipient (9) est pourvu d'un capuchon cassable, ouvrable ou perforable (60), le dispositif de couplage et de perforation (8) comprenant un premier élément couplé de manière coulissante à un deuxième élément (22) et étant mobile entre la position de couplage la bouteille (9 ) et la position de perforation du capuchon (60) de la bouteille (9), dans lequel le premier élément comprenant un anneau (23) à partir duquel partent verticalement au moins deux rabats (24) qui sont équipés d'encoches (25, 26), qui sont adaptés au maintien de la bouteille (9) en position de couplage, et en ce que la fermeture ouvrable (7) est surmontée d'une base circulaire (28), à partir de laquelle partent des paires d'autres rabats (27), qui sont à leur tour équipés d'encoches (52) adaptées au maintien de la bouteille (9) pour s'adapter à la position de perforation du capuchon (60), et qui se bifurquent périmétriquement et verticalement l'un à côté de l'autre, dans lequel ledit deuxième élément (22) présentant des espaces (30) entre les paires d'autres rabats (27) conformément avec les au moins deux rabats (24), les espaces (30) sont adaptés pour recevoir les au moins deux rabats (24) avec la bouteille (9) dans la position de perforation du capuchon (60).

10. Le kit selon l'une quelconque des revendications 1 à 9, dans lequel l'au moins une substance pharmaceutiquement active (70) est destinée à être utilisée dans le traitement de cancers et/ou de tumeurs traitables par irrigation et/ou perfusion, de préférence dans le traitement du cancer et/ou des tumeurs sélectionnées dans le groupe constitué d'un cancer/tumeur du péritoine, d'un cancer/tumeur des voies urinaires, en particulier d'un cancer de la vessie, d'un cancer de l'ovaire, d'un cancer/tumeur du côlon et, de préférence, d'un cancer/tumeur du péritoine; et/ou dans le traitement du cancer et/ou des tumeurs choisies dans le groupe constitué notamment du cancer du côlon, du cancer du col de l'utérus, du cancer de l'utérus, du cancer de la vessie, du cancer du poumon, du cancer de l'œsophage, du cancer de l'estomac, du cancer du pancréas, du cancer péritonéal et du cancer de la prostate comme ainsi que leurs formes métastatiques.

11. Le kit selon l'une quelconque des revendications 1 à 9, dans lequel l'au moins une substance pharmaceutiquement active (70) est destinée à être utilisée pour une administration au moyen d'une thérapie par aérosol sous pression (PAT) dans un organe creux ou dans une cavité corporelle d'un patient pour une efficacité (thérapeutique) non systémique, de préférence pour une efficacité (thérapeutique) locale et/ou topique dans un organe creux ou dans une cavité corporelle d'un patient.

12. Le kit selon l'une quelconque des revendications 1 à 9, dans lequel l'au moins une substance pharmaceutiquement active (70) est choisie parmi
(a) le groupe constitué des substances antitumorales, anticancéreuses, cytostatiques, antinécrotiques, antimicrobiennes, antifongiques, antibactériennes, antivirales, antiseptiques, antibiotiques ou une combinaison de celles-ci; et/ou
(b) le groupe constitué de substances anticancéreuses, antitumorales, cytostatiques ou d'une combinaison de celles-ci.

13. Le kit selon la revendication 12, dans lequel l'au moins une substance pharmaceutiquement active (70) est choisie parmi le groupe constitué des substances anticancéreuses, antitumorales et cytostatiques ou une combinaison de celles-ci est sélectionnée dans le groupe constitué de l'aflibercept, de l'amsacrine, de l'alemtuzumab, du trioxyde d'arsenic, asparaginase, azacitidine, chlorhydrate de bendamustine, bevacizumab, bléomycine, blinatumomab, bortézomib, busulfan, cabacitaxel, carboplatine, carfilzomib, carmustine, 2 CdA/cladribine, cetuximab, cidofovir, cisplatine, clofarabine, cyclophosphamide, cytarabine, dacarbacin, dactinomycine, daratumab, daunorubicine , décitabine, docétaxel, doxorubicine, doxorubicine liposomale, mésilate d'éribuline, épirubicine, étoposide, fludarabine, 5-fluorouracile, foscarnet, gémicitabine, glancyclovir, idarubicine, ifosfamide, ipilmumab, irinotécan, melphalan, méthotrexate, mifamurtide, mitomycine, mitoxantrone, larabine, umab , obinituzumab , ofatumumab, oxaliplatin, paclitaxel, panitumab, pemetrexed, pertuzumab, prembolizumab, pamucirumab, pituximab, pomidepsin, thiotepa, topotecan, trabectedin, trastuzumab, trastuzumab-emtansine, treosulfan, vinblastine, vincristine, vindesine, vinflunine, vinorelbine.

14. Le kit selon la revendication 12, dans lequel l'au moins une substance pharmaceutiquement active (70) est choisie parmi l'un des éléments suivants:
(c) une combinaison de doxorubicine, de cisplatine, d'oxaliplatine, de topotécan et de paclitaxel;
(d) une combinaison de pémétrexed, de bendamustine, de topotécan et de carboplatine;
(e) une combinaison d'irinotécan et de docétaxel;
(f) une combinaison de doxorubicine, de mitomycine et d'épirubicine;
(g) une combinaison de docétaxel, d'oxaliplatine, de paclitaxel et de vinorelbine.
